# EUROPEAN PATENT SPECIFICATION

(11) **EP 2 988 763 B1**
(45) Date of publication and mention of the grant of the patent: **12.07.2023**
(21) Application number: 14788557.8
(22) Date of filing: 22.04.2014
(51) Int. Cl.: A61K 35/74, A61K 36/06, A61K 35/747, A61P 31/10, A61P 31/04, A01N 63/20, A01N 63/30, A01P 1/00, A01P 3/00

(54) **BACTERIAL STRAIN HAVING ANTIMICROBIAL ACTIVITY USED ON PLANTS**
AUF PFLANZEN VERWENDETER BAKTERIENSTAMM MIT ANTIMIKROBIELLER AKTIVITÄT
SOUCHE BACTÉRIENNE POSSÉDANT UNE ACTIVITÉ ANTIMICROBIENNE UTILISÉE SUR DES PLANTES

(30) Priority: 23.04.2013 US 201361815038 P
(43) Date of publication of application: 02.03.2016
(73) Proprietor: Terragen Holdings Limited, Melbourne VIC 3000 (AU)
(72) Inventor: FINLAYSON, Wayne, Queensland 4573 (AU); JURY, Karen, Lennox Head, New South Wales 2478 (AU)
(74) Representative: Potter Clarkson
(86) International application number: PCT/AU2014/050019
(87) International publication number: WO 2014/172758

(56) References cited:
- WO-A1-94/19950
- WO-A1-97/29644
- WO-A1-97/36603
- WO-A1-2008/049230
- WO-A1-2012/136830
- WO-A1-2013/063658
- WO-A1-2013/147426
- WO-A1-2013/174792
- WO-A2-00/71139
- WO-A2-2006/136420
- CN-A- 102 851 232
- KR-A- 20100 005 519
- KR-A- 20120 022 291
- US-A- 4 591 499
- ASAHARA, T. ET AL.: 'Protective Effect of Lactobacillus casei Strain Shirota Against Lethal Infection with Multi-drug Resistant Salmonella enterica serovar Typhimurium DT104 in Mice' JOURNAL OF APPLIED MICROBIOLOGY vol. 110, no. 1, January 2011, pages 163 - 173, XP055066572
- ROUSE, S. ET AL.: 'Bioprotective Potential of Lactic Acid Bacteria in Malting and Brewing' JOURNAL OF FOOD PROTECTION vol. 71, no. 8, August 2008, pages 1724 - 1733, XP008181082
- TRIAS, R. ET AL.: 'Lactic Acid Bacteria from Fresh Fruit and Vegetables as Biocontrol Agents of Phytopathogenic Bacteria and Fungi' INTERNATIONAL MICROBIOLOGY vol. 11, no. 4, December 2008, pages 231 - 236, XP009165082

## Description

### Field of the Art

The present disclosure relates to a bacterial strain that is used in a method for inhibiting microbial plant pathogens.

### Background

All plants are susceptible to attack by microorganisms, such as bacteria and fungi. In the case of fruits, vegetables, and agricultural and horticultural crops microbial pathogens and diseases caused by them can result in significant crop damage, loss of yield and economic losses both preharvest and postharvest. Diseases caused by microbial pathogens can also lead to decreased shelf-life of produce, and to higher costs for consumers.

Many fungi are known plant pathogens causing many diseases that harm or destroy crops worldwide. Most plant pathogenic fungi are ascomycetes (including *Fusarium* spp., *Thielaviopsis* spp., *Botrytis* spp., *Verticillium* spp., and *Magnaporthe* spp.) and basidiomycetes (including *Rhizoctonia* spp., *Puccinia* spp. and *Armillaria* spp.). Of particular concern are fungi of the genus *Fusarium,* filamentous fungi widely distributed in soil. For example, a number of *Fusarium* species such as *Fusarium oxysporum* affect plants including tomatoes, melons, ginger, bananas and legumes with a wilt disease (Fusarium Wilt) causing symptoms such as vascular wilt, necrosis, premature leaf drop and stunting of growth. *Fusarium*dry rot of potatoes is caused by several species of the *Fusarium* genus. *Fusarium* dry rot is an economically important problem in potatoes, both in the field and in storage, and is one of the leading causes of postharvest potato losses. In bananas *Fusarium* is the causal agent of the highly destructive Panama disease. Once a plantation is infected there is no cure. *Fusarium oxysporum* is an imperfect asexual fungus that spreads by means of three types of spores: microconidia, macroconidia, and chlamydospores. Once *Fusarium oxysporum* is established in soil it is known to be extremely difficult to eradicate because chlamydospores can remain dormant and infect the soil for many years. Due to the ineffectiveness of fungicides, the only currently available response is soil sterilization, which is not cost effective.

Plant pathogenic bacteria also cause many damaging and economically significant diseases in plants. Examples include species of *Erwinia, Pectobacterium, Pantoea, Agrobacterium, Pseudomonas, Ralstonia, Burkholderia, Acidovorax, Xanthomonas, Clavibacter, Streptomyces, Xylella, Spiroplasma,* and *Phytoplasma.* Plant pathogenic bacteria cause a range of symptoms including galls and overgrowths, wilts, leaf spots, specks and blights, soft rots, as well as scabs and cankers.

Potato scab is a disease that infects potato tubers as well as other root crops such as radish, beet, carrot and parsnips. It causes unsightly necrotic lesions on the tuber surface resulting in huge economic losses. The primary causal pathogen is *Streptomyces scabies* found in the soil of potato growing regions worldwide, a Gram positive, aerobic filamentous bacteria producing grey mycelia on most solid media. The vegetative filaments break off to form spores enabling the bacteria to survive long periods of time and spread via water, wind and soil. It is able to survive long periods of time, even years, in the soil surviving on decaying plant material, as well as surviving passage through animal digestive tracts. Various approaches have been used to reduce disease severity, however presently no effective control is available for potato scab.

Fungicides and other pesticides applied to plants to combat pathogenic microorganisms and to treat or prevent diseases caused by such pathogens are typically chemical in nature (often synthetic and non-naturally occurring). These can be expensive to manufacture and bring with them unwanted side effects, including toxicity to animals, and environmental concerns. There is a clear and continuing need for the development of alternative approaches. Biocontrol agents and compositions are an attractive alternative, being safer, more biodegradable, and less expensive to develop. US 4591499 A relates a method for treating mastitis in ruminants by the administration of non-pathogenic *Lactobacilli.*
CN 102 851 232 A relates to *a Lactobacillus* strain which is *Lactobacillus parafarraginis* ZH1, and an application thereof. WO 94/19950 A1 relates to a biocontrol agent which includes or is derived from a sourdough starter formulation comprising a mixed culture of the yeast component, a bacterial component and a substrate for the mixed culture. KR 2012 0022291 A relates to a feed additive for promoting the growth of domestic animals to ferment biotite using water, microorganisms, and nutrients selected from wheat bran, rice bran, or molasses. WO 97/36603 relates to a *Lactobacillus casei* strain which is effective in the treatment of fungal infections. WO 2008/049230 A1 relates to an isolated nucleic acid molecule encoding a protein inhibitory to *Sclerotinia* spp. Salvetti et al. (2012), Antimicro. 4: 217-226 relates to the taxonomic classification of bacteria of the genus *Lactobacillus.*

### Summary of the Disclosure

The present invention is defined in the accompanying claims and relates to a method of treating or preventing infection of a plant by a fungal or bacterial pathogen. Embodiments of the invention as described in the following numbered paragraphs:
(1) A method of treating or preventing infection of a plant by a fungal or bacterial pathogen, the method comprising administering to the plant, or otherwise exposing the plant to, an effective amount of a composition comprising *Lactobacillus parafarraginis* strain Lp18 deposited with National Measurement Institute, Australia on 27 October 2011 under Accession Number V11/022945, or a culture supernatant or cell free filtrate derived from culture media in which said *Lactobacillus parafarraginis* strain has been cultured, wherein the fungal or bacterial pathogen is selected from *Pseudocercospora macadamiae, Botrytis cinerea, Streptomyces scabies,* and *Pseudomonas savastanoi.*
(2) The method according to paragraph 1, wherein the *Lactobacillus parafarraginis* is encapsulated.
(3) The method according to paragraph 1 or paragraph 2, wherein the plant is a crop species.

The present disclosure also provides a method for treating or preventing infection of a subject by a microbial pathogen, the method comprising administering to the subject, or otherwise exposing the subject to, an effective amount of a composition comprising at least one strain of *Lactobacillus* selected from *Lactobacillus parafarraginis, Lactobacillus buchneri, Lactobacillus rapi* and *Lactobacillus zeae,* or a culture supernatant or cell free filtrate derived from culture media in which said strain has been cultured, wherein the subject matter falling outside the scope of the invention as described above is provided for illustrative purposes.

The present disclosure also provides a method for treating or preventing a disease in a subject caused by, or associated with, a microbial pathogen, the method comprising administering to the subject, or otherwise exposing the subject to, an effective amount of a composition comprising at least one strain of *Lactobacillus* selected from *Lactobacillus parafarraginis, Lactobacillus buchneri, Lactobacillus rapi* and *Lactobacillus zeae,* or a culture supernatant or cell free filtrate derived from culture media in which said strain has been cultured, wherein the subject matter falling outside the scope of the invention as described above is provided for illustrative purposes.

In accordance with the above, exposing the subject to the composition may comprise directly or indirectly exposing the subject to the composition. By way of example, where the subject is a plant, the plant may be exposed to the composition by application of the composition to a part of the plant or to the soil into which the plant is growing or is to be planted. Also by way of example, where the subject is an animal, the animal may be exposed to the composition by application of the composition to pasture or other grass (or soil on which the pasture or other grass is grown) on which the animal feeds.

The method may further comprise administering to the subject, or otherwise exposing the subject to, an effective amount of one or more antimicrobial agents.

Provided herein but not claimed is a method for inhibiting the growth of a microorganism, the method comprising exposing the microorganism, or an environment colonised by or capable of being colonised by the microorganism, to an effective amount of a composition comprising at least one strain of *Lactobacillus* selected from *Lactobacillus parafarraginis, Lactobacillus buchneri, Lactobacillus rapi* and *Lactobacillus zeae,* or a culture supernatant or cell free filtrate derived from culture media in which said strain has been cultured.

In exemplary options the subject is a plant. In further exemplary options, the subject is a plant and the environment is soil, plant roots and/or plant foliage. Soil may be treated with the composition prior to planting of the plant, at the time of planting or after planting. Similarly, plant roots may be treated with the composition prior to planting of the plant, at the time of planting or after planting.

The method may comprise one treatment or multiple treatments of the environment or the subject with the composition.

The method may further comprise exposing the microorganism to an effective amount of one or more antimicrobial agents.

Provided herein but not claimed is a biocontrol composition for treating or preventing infection of a subject by a microbial pathogen, the composition comprising at least one strain of *Lactobacillus* selected from *Lactobacillus parafarraginis, Lactobacillus buchneri, Lactobacillus rapi* and *Lactobacillus zeae,* or a culture supernatant or cell free filtrate derived from culture media in which said strain has been cultured.

Also disclosed herein but not claimed is a biocontrol composition for the treatment or prevention of a disease in a subject caused by, or associated with, infection of the subject by a microbial pathogen, the composition comprising at least one strain of *Lactobacillus* selected from *Lactobacillus parqfarraginis, Lactobacillus buchneri, Lactobacillus rapi* and *Lactobacillus zeae,* or a culture supernatant or cell free filtrate derived from culture media in which said strain has been cultured.

Also disclosed herein but not claimed is a composition for inhibiting the growth of a microorganism, the composition comprising at least one strain of *Lactobacillus* selected from *Lactobacillus parafarraginis. Lactobacillus buchneri. Lactobacillus rapi* and *Lactobacillus zeae.* or a culture supernatant or cell free filtrate derived from culture media in which said strain has been cultured.

In addition to the components of compositions described in the above aspects, compositions disclosed herein may comprise one or more strains of *Lactobacillus diolivorans* (N3) deposited with the National Measurement Institute, Australia on 14 December 2012 under Accession Number V12/022847, *Lactobacillus parafarraginis* (N11) deposited with the National Measurement Institute. Australia on 14 December 2012 under Accession Number V12/022848, *Lacwbacillus brevis* (TD) deposited with the National Measurement Institute, Australia on 14 December 2012 under Accession Number Vl2/022851, *Lactobacillus paracasei,* strain designated 'T9' deposited with the National Measurement Institute, Australia on 14 December 2012 under Accession Number Vl2/022849, *Lactobacillus casei* and strain designated herein 'TB' deposited with the National Measurement Institute, Australia on 14 December 2012 under Accession Number V 12/022850; or a culture supernatant or cell free filtrate derived from culture media in which one or more of these strains have been cultured.

Also provided herein but not claimed is the use of one or more strains of *Lactobacillus,* wherein the *Lactobacillus* is selected *from Lactobacillus parqfarraginis. Lactobacillus buchneri. Lactobacillus rapi* and *Lactobacillus zeae,* for the manufacture of a composition for treating or preventing infection of a subject by a microbial pathogen.

Also provided herein but not claimed is the use of one or more strains of *Lactobacillus,* wherein the *Lactobacillus* is selected from *Lactobacillus parafarraginis. Lactobacillus buchneri. Lactobacillus rapi* and *lactobacillus zeae,* for the manufacture of a composition for treating or preventing a disease in a subject caused by, or associated with, infection of the subject by a microbial pathogen.

Also provided herein but not claimed is the use of one or more strains of *Lactobacillus,* wherein the *Lactobacillus* is selected from *Lactobacillus parafarraginis, Lactobacillus buchneri. Lactobacillus rapi* and *Lactobacillus zeae,* for the manufacture of a composition for inhibiting the growth of a microorganism.

Also provided herein but not claimed is the use of one or more strains of *Lactobacillus,* wherein the Lactobacillus is selected from *Lactobacillus parafarraginis, Lactobacillus buchneri. Lactobacillus rapi, Lactobacillus zeae. Lactobacillus paracasei,* strain designated herein T9' deposited with the National Measurement Institute, Australia on 14 December 2012 under Accession Number V12/022849, *Lactobacillus easel* and strain designated herein "TB' deposited with the National Measurement Institute, Australia on 14 December 2012 under Accession Number V12/022850, for the manufacture of a composition for treating or preventing infection of a subject by a microbial pathogen.

Also provided herein but not claimed is the use of one or more strains of *Lactobacillus,* wherein the Lactobacillus is selected from *Lactobacillus parafarraginis, Lactobacillus buchneri. Lactobacillus rapi. Lactobacillus zeae, Lactobacillus paracasei,* strain designated herein 'T9' deposited with the National.Measurement Institute, Australia on 14 December 2012 under Accession Number V 12/022849. *Lactobacillus casei* and strain designated herein 'TB' deposited with the National Measurement Institute, Australia on 14 December 2012 under Accession Number V 12/022850, for the manufacture or a composition for treating or preventing a disease in a subject caused by. or associated with, infection of the subject by a microbial pathogen.

Also provided herein but not claimed is the use of one or more strains of *Lactobacillus,* wherein the Lactobacillus is selected from *Lactobacillus parafarraginis, Lactobacillus buchneri. Lactobacillus rapi, Lactobacillus zeae, Lactobacillus paracasei,* strain designated herein "T9" deposited with the National Measurement Institute Australia on 14 December 2012 under Accession Number VJ 2/022849, *Lactobacillus casei* and strain designated herein 'TB' deposited with the National Measurement Institute, Australia on 14 December 2012 under Accession Number V12/022850, for the manufacture of a composition for inhibiting the growth of a microorganism.

Also provided herein but not claimed is the use of one or more of the following strains for the manufacture of a composition for treating or preventing infection of a subject by a microbial pathogen, for treating or preventing a disease in a subject caused by, or associated with, infection of the subject by a microbial pathogen, or for inhibiting the growth of a microorganism: *Lactobacillus diolivorans* (N3) deposited with the National Measurement Institute, Australia on 14 December 2012 under Accession Number V12/022847, *Lactobacillus parafarraginis* (N11) deposited with the National Measurement Institute, Australia on 14 December 2012 under Accession Number V12/022848, *Lactobacillus brevis* (TD) deposited with the National Measurement Institute, Australia on 14 December 2012 under Accession Number V12/022851, *Lactobacillus paracasei,* strain designated herein 'T9' deposited with the National Measurement Institute, Australia on 14 December 2012 under Accession Number V12/022849, *Lactobacillus casei* and strain designated herein 'TB' deposited with the National Measurement Institute, Australia on 14 December 2012 under Accession Number V12/022850.

The following disclosure relates to all of the above aspects and options.

In accordance with the claimed invention, the *Lactobacillus parafarraginis* strain is *Lactobacillus parafarraginis* Lp18 deposited with National Measurement Institute, Australia on 27 October 2011 under Accession Number V11/022945.

As disclosed herein but not claimed, the *Lactobacillus buchneri* strain may be *Lactobacillus buchneri* Lb23. In a particular option the *Lactobacillus buchneri* strain is *Lactobacillus buchneri* Lb23 deposited with National Measurement Institute, Australia on 27 October 2011 under Accession Number V11/022946.

As disclosed herein but not claimed, the *Lactobacillus rapi* strain may be *Lactobacillus rapi* Lr24. In a particular option the *Lactobacillus rapi* strain is *Lactobacillus rapi* Lr24 deposited with National Measurement Institute, Australia on 27 October 2011 under Accession Number V11/022947.

As disclosed herein but not claimed, the *Lactobacillus zeae* strain may be *Lactobacillus zeae* Lz26. In a particular option the *Lactobacillus zeae* strain is *Lactobacillus zeae* Lz26 deposited with National Measurement Institute, Australia on 27 October 2011 under Accession Number V11/022948.

The composition may further comprise a strain of *Acetobacter fabarum.* The *Acetobacter fabarum* strain may be *Acetobacter fabarum* Afl5. In a particular option the *Acetobacter fabarum* strain is *Acetobacter fabarum* Afl5 deposited with the National Measurement Institute, Australia on 27 October 2011 under Accession Number V11/022943.

The composition may further comprise a yeast. The yeast may be a strain of *Candida ethanolica.* The *Candida ethanolica* strain may be *Candida ethanolica* Ce31. In a particular option the *Candida ethanolica* strain is *Candida ethanolica* Ce31 deposited with the National Measurement Institute, Australia on 27 October 2011 under Accession Number V11/022944.

The composition may comprise two or more of said *Lactobacillus* species, three of said *Lactobacillus* species or all of said *Lactobacillus* species. The composition may represent a symbiotic combination of two or more or three or more of said *Lactobacillus* species.

One or more of the strains in the composition may be encapsulated. Where multiple strains are encapsulated, the strains may be individually encapsulated or combined in a single encapsulation.

The composition may further comprise one or more antimicrobial agents.

The subject is a plant in accordance with the claimed invention. For example, the plant may be an agricultural crop species, a horticultural crop species or a crop species for fuel or pharmaceutical production. In another exemplary option not falling within the scope of the claimed invention the subject is an animal, particularly a non-human animal, such as a milk-producing mammal (for example a cow or goat).

The microbial pathogen may be a causative agent of a plant disease or animal disease. In exemplary options, the disease may be selected from a rot, wilt, rust, spot, blight, canker, mildew, mould, gall, scab or mastitis.

The microbial pathogen is a fungus or bacteria in accordance with the claimed invention.

In accordance with the claimed invention, the fungal pathogen is selected from *Pseudocercospora macadamiae* and *Botrytis cinerea.* In some other examples, that may not fall within the scope of the invention, the fungus may be selected from a *Fusarium* sp., a *Pseudocercospora* sp., a *Phialemonium* sp, a *Botrytis* sp. or a *Rhizoctonia* sp. The *Fusarium* sp. may be *Fusarium oxysporum,* such as *Fusarium oxysporum* f. sp. *zingiberi, Fusarium oxysporum*f. sp. *niveum* or *Fusarium oxysporum* f. sp. *cubense.* The *Pseudocercospora* sp. may be *Pseudocercospora macadamiae.* The *Phialemonium* sp. may be *Phialemonium dimorphosporum.* The *Botrytis* sp. may be *Botrytis cinerea.* The *Rhizoctonia* sp. may be *Rhizoctoniasolani.* The skilled addressee will appreciate that this list is not exhaustive. Additional suitable fungal species are disclosed hereinbelow.

The bacteria may be Gram positive or Gram negative. In accordance with the claimed invention, the bacterial pathogen is selected from *Streptomyces scabies* and *Pseudomonas savastani.* In some other examples that may not fall within the scope of the invention, the bacteria may be a *Streptomyces* sp., a *Staphylococcus* sp., an *Escherichia* sp., a *Pseudomonas* sp., a *Pantoea* sp. or a *Streptococcus* sp. The *Streptomyces* sp. may be *Streptomyces scabies.* The *Staphylococcus* sp. may be *Staphylococcus aureus.* The *Streptococcus* sp. may be *S. uberis.* The *Escherichia* sp. may be *Escherichia coli.* The *Pseudomonas* sp. may be *Pseudomonas savastani.* The *Pantoea* sp. may be *Pantoea agglomerons.* The skilled addressee will appreciate that this list is not exhaustive. Additional suitable bacterial species are disclosed hereinbelow.

### Brief Description of the Drawings

Aspects and embodiments of the present disclosure are described herein, by way of non-limiting example only, with reference to the following drawings.

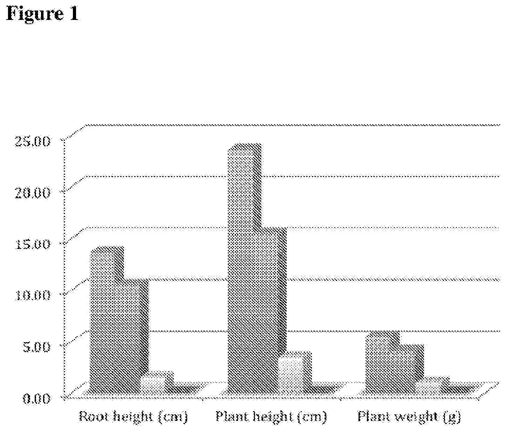

**Figure 1****.** Root height, plant height and plant weight of watermelon plants following three weeks of treatment as described in Example 3. For each parameter, the bars represent, from left to right, watermelon seedlings from experiments A, B, C and D.

### Detailed Description

Unless defined otherwise, all technical and .scientific terms used herein have the same meaning as commonly understood by those of ordinary skill in the art to which the disclosure belongs. Although any methods and materials similar or equivalent to those described herein can be used in the practice or testing of the present disclosure, typical methods and materials are described.

The articles "a" and "an" are used herein to refer to one or to more than one (*i*. *e*., to at least one) of the grammatical object of the article. By way of example, "an element" means one element or more than one element.

In the context of this specification, the term "about," is understood to refer to a range of numbers that a person of skill in the art would consider equivalent to the recited value in the context of achieving the same function or result

Throughout this specification and the claims which follow, unless the context requires otherwise, the word "comprise", and variations such as "comprises" or "comprising", will be understood to imply the inclusion of a stated integer or step or group of integers or steps but not the exclusion of any other integer or step or group of integers or steps.

As used herein the term "antimicrobial agent" refers to any agent that, alone or in combination with another agent, is capable of killing or inhibiting the growth of one or more species of microorganism. Antimicrobial agents include, but are not limited to, antibiotics, detergents, surfactants, agents that induce oxidative stress, bacteriocins and antimicrobial enzymes (e.g. lipases, pronases and lyases) and various other proteolytic enzymes and nucleases, peptides and phage. Reference to an antimicrobial agent includes reference to both natural and synthetic antimicrobial agents.

As used herein the term "exposing" means generally bringing into contact with. Exposure of a subject to a composition or agent as described herein includes administration of the composition or agent to the subject, or otherwise bringing the composition or agent into contact with the subject, whether directly or indirectly.For example, exposing a subject to a composition or agent may include applying or administering the composition or agent to an environment inhabited by the subject or to a feed, liquid or other nutrient composition to be administered by the subject. In the present disclosure the terms "exposing", "administering" and "contacting" and variations thereof may, in some contexts, be used interchangeably.

The term "inhibiting" and variations thereof such as "inhibition" and "inhibits" as used herein in relation to microbial growth refers to any microcidal or microstatic activity of a composition or agent. Such inhibition may be in magnitude and/or be temporal or spatial in nature. Inhibition of the growth of bacteria or fungi by a composition or agent can be assessed by measuring microbial growth in the presence and absence of the composition or agent. The microbial growth may be inhibited by the composition or agent by at least or about 10%, 15%, 20%. 25%, 30%, 35%, 40%, 45%, 50%, 55%, 60%, 65%, 70%. 75%, 80%, 85%, 90%. 95% or more compared to the growth of the same microbe that is not exposed to the composition or agent.

The term "subject" as used herein refers to any plant or animal infected or suspected of being infected by, or susceptible to infection front, a microbial pathogen. Plants include, without limitation, plants that produce fruits, vegetables, grains, tubers, legumes. flowers, and leafs or any other economically or environmentally important plant. Thus the plant may be a crop species. The term "crop" as used herein refers to any plant grown to be harvested or used for any economic purpose, including for example human foods, livestock fodder, fuel or pharmaceutical production (e.g. poppies). Animals include, for example, mammals, birds, fish, reptiles, amphibians, and any other vertebrates or invertebrates, such as those of economic, environmental, and/or other significant importance. Mammals include, but are not limited to, livestock and other farm animals (such as cattle. goats. sheep. horses, pigs and chickens), performance animals (such as racehorses), companion animals (such as cats and dogs), laboratory test animals and humans.

As used herein, the term "effective amount" refers to an amount of microbial inoculant or fertilizer composition applied to a given area of soil or vegetation that is sufficient to effect one or more beneficial or desired outcomes, for example, in terms of plant growth rates, crop yields, or nutrient availability in the soil. An "effective amount" can be provided in one or more administrations. The exact amount required will vary depending on factors such as the identity and number of individual strains employed, the plant species being treated, the nature and condition of the soil to be treated, the exact nature of the microbial inoculant or fertilizer composition to be applied, the form in which the inoculant or fertilizer is applied and the means by which it is applied, and the stage of the plant growing season during which application takes place. Thus, it is not possible to specify an exact "effective amount". However, for any given case, an appropriate "effective amount" may be determined by one of ordinary skill in the art using only routine experimentation.

As used herein the terms "treating", "treatment" and the like refer to any and all applications which remedy, or otherwise hinder, retard, or reverse the progression of, an infection or disease or at least one symptom of an infection or disease, including reducing the severity of an infection or disease. Thus, treatment does not necessarily imply that a subject is treated until complete elimination of the infection or recovery from a disease. Similarly, the terms "preventing", "prevention" and the like refer to any and all applications which prevent the establishment of an infection or disease or otherwise delay the onset of an infection or disease.

The term "optionally" is used herein to mean that the subsequently described feature may or may not be present or that the subsequently described event or circumstance may or may not occur. Hence the specification will be understood to include and encompass options in which the feature is present and options in which the feature is not present, and options in which the event or circumstance occurs as well as options in which it does not.

Provided herein, but not necessarily falling within the claimed invention unless as described above, are methods for treating or preventing infection of a subject by a microbial pathogen, comprising administering to the subject, or otherwise exposing the subject to, an effective amount of a composition comprising at least one strain of *Lactobacillus* selected from *Lactobacillus parafarraginis, Lactobacillus buchneri, Lactobacillus rapi* and *Lactobacillus zeae.*

Also provided are methods for treating or preventing a disease in a subject caused by, or associated with, a microbial pathogen, the method comprising administering to the subject, or otherwise exposing the subject to, an effective amount of a composition comprising at least one strain of *Lactobacillus* selected from *Lactobacillus parafarraginis, Lactobacillus buchneri. Lactobacillus rapi* and *Lactobacillus zeae,* wherein the embodiments falling outside the scope of the claimed invention are provided as examples.

Further provided herein but not forming part of the claimed invention are methods for inhibiting the growth of a microorganism, the method comprising exposing the microorganism, or an environment colonised by or capable of being colonised by the microorganism. to an effective amount or a composition comprising at least one strain of *Lactobacillus* selected from *Lactobacillus parafarraginis, Lactobacillus buchneri. Lactobacillus rapi* and *Lactobacillus zeae.*

Also provided herein arc methods for treating or preventing infection of a subject by a microbial pathogen, comprising administering to the subject or otherwise exposing the subject to, an effective amount of a composition comprising at least one strain of *Lactobacillus* selected from *Lactobacillus parafarraginis. Lactobacillus buchneri. Lactobacillus rapi. Lactobacillus zeae, Lactobacillus casei, Lactobacillus paracasei,* strain designated herein 'T9' deposited with the National Measurement Institute, Australia on 14 December 2012 under Accession Number Vl2/022849, and strain designated herein 'TB' deposited with the National Measurement Institute, Australia on 14 December 2012 under Accession Number V12/022850 wherein the embodiments falling outside the scope of the claimed invention are provided as examples.

Also provided arc methods for treating or preventing a disease in a subject caused by, or associated with, a microbial pathogen, the method comprising administering to the subject or otherwise exposing the subject to, an effective amount of a composition comprising at least one strain of *Lactobacillus* selected from *Lactobacillus parafarraginis. Lactobacillus buchneri. Lactobacillus rapi. Lactobacillus zeae, Lactobacillus casei. Lactobacillus paracasei,* strain designated herein 'T9' deposited with the National Measurement Institute, Australia on 14 December 2012 under Accession Number Vl2/022849, and strain designated herein "TB' deposited with the National Measurement Institute Australia on 14 December 2012 under Accession Number V 12/022850 wherein the embodiments falling outside the scope of the claimed invention are provided as examples.

Further provided herein but not forming part of the claimed invention are methods for inhibiting the growth of a microorganism, the method comprising exposing the microorganism, or an environment colonised by or capable of being colonised by the microorganism, to an effective amount of a composition comprising at least one strain of *Lactobacillus* selected from *Lactobacillus parqfarraginis, Lactobacillus buchneri. Lactobacillus rapi. Lactobacillus zeae, Lactobacillus casei, Lactobacillus paracasei,* strain designated herein 'T9' deposited with the National Measurement Institute, Australia on 14 December 2012 under Accession Number V12/022849, and strain designated herein 'TB' deposited with the National Measurement Institute, Australia on 14 December 2012 under Accession Number V12/022850.

Novel biocontrol compositions are also provided for treating and preventing infections caused by microbial pathogens, and diseases caused by, or associated with, such infections, and for inhibiting the growth of microorganisms, but do not form part of the claimed invention.

In accordance with the present disclosure, the microbial pathogen or microorganism may be a fungal or bacterial pathogen capable of infecting and/or causing disease in any plant or animal species. Methods and compositions of the present disclosure therefore find application in the treatment and prevention of fungal diseases of plants and animals, such as rots, wilts, rusts, spots, blights, cankers, mildews and moulds. Methods and compositions of the present disclosure also find application in the treatment and prevention of bacterial diseases of plants and animals, including galls, scabs and other diseases such as mastitis.

In exemplary options, methods and compositions disclosed herein may be employed in the treatment and prevention of fungal diseases selected from Fusarium dry rot, Fusarium wilt, black dot, late blight, black scurf, Rhizoctonia, pink rot, target spot, Panama disease, stripe rust (yellow rust), soft rot, stem rust (black rust), grey mould, Phytophthora heart rot, smut, Phytophthora rot, peanut rust, Rhizoctonia stem rot, rhizome rot, fungal husk spot, trunk canker, white root rot, verticillium wilt and ginger yellows, and of infections by the causative agents of these diseases. Where the subject is a plant, the plant affected by the disease may be, for example, a food crop (for humans or other animals) such as any fruit, vegetable, nut, seed or grain producing plant. Exemplary crop plants include, but are not limited to, tubers and other below-ground vegetables (such as potatoes, beetroots, radishes, carrots, onions, etc.), ground-growing or vine vegetables (such as pumpkin and other members of the squash family, beans, peas, asparagus, etc.), leaf vegetables (such as lettuces, chard, spinach, alfalfa, etc.), other vegetables (such as tomatoes, brassica including broccoli, avocadoes, etc.), fruits (such as berries, olives, stone fruits including nectarines and peaches, tropical fruits including mangoes and bananas, apples, pears, watermelon, mandarins, oranges, mandarins, kiwi fruit, coconut, etc.), cereals (such as rice, maize, wheat, barley, millet, oats, rye etc.), nuts (such as macadamia nuts, peanuts, brazil nuts, hazel nuts, walnuts, almonds, etc.), and other economically valuable crops and plants (such as garlic, ginger, sugar cane, soybeans, sunflower, canola, sorghum, pastures, turf grass, etc).

Where the subject is an animal, in particular exemplary options not falling within the scope of the claims, the animal may be exposed to a composition disclosed herein indirectly by application of the composition to pasture, grass or other plant on which the animal feeds. In such options, exemplary animals are dairy cattle.

In exemplary options, methods and compositions disclosed herein may be employed in the treatment and prevention of bacterial diseases selected from common scab, bacterial spot, bacterial speck, potato scab, bacterial soft rot, crown gall disease and mastitis, and of infections by the causative agents of these diseases.

Fungal pathogens against which methods and compositions disclosed herein find application include, but are not limited to, *Fusarium spp.* such *asFusarium oxysporum* and special forms thereof including *Fusarium oxysporum f. sp. zingiberi, Fusarium oxysporum* f.sp. *niveum,* and *Fusarium oxysporum* f.sp. *cubense; Collectotrichum coccodes; Phytophthora spp.* such as *Phytophthora infestans, Phytophthora erythroseptica, Phytophthora cinnamomi; Rhizoctonia solani; Corynespora cassiicola; Puccinia spp.* such as *Puccinia arachidus, Puccinia striiformis, Puccinia graminis f. sp. tritici; Botrytis cinerea; Rhizoctonia; Pythium myriotylum; Psuedocercospora macadamiae; Rosellinia necartrix; Verticillum spp.* such as *Verticillum deliliae; Phialemonium dimorphosporum; Thielaviopsis spp.; Magnaporthe grisea.*

Bacterial pathogens against which methods and compositions disclosed herein find application include, but are not limited to, *Streptomyces scabies; Xanthomonas* spp. such as *Xanthomonascampestris pv. Vesicatoria; Pseudomonas spp.* such as *P. savastanoi, P. syringae, P. aeruginosa, and P. fluorescens; E. coli; Listeriamonocytogenes; Staphylococcus spp.* such as *S. aureus; Streptococcus spp.* such as *S. uberis; Agrobacterium tumefaciens; Burkholderia cepacia; Erwinia carotovora; Erwinia chrysanthemi; Pantoea agglomerons; Ralstonia solanacearum; Pantoea stewartii; Aeromonas hydrophila; Vibrio spp.* such as *Y. anguillarum, V. harveyi, Y. cholera,* and *Y. parahaemoliticus; Actinobacillus pleuropneumoniae;Chromobacter violaceum; Coxiella burnetti;Francisella tularensis; Haemophilus influenza; Pasteurella multocida; Shigella flexneri; Salmonella typhi; Salmonella typhimurium; Yersinia pestis;* and *Yersinia pseudotuberculosis.*

Those skilled in the art will recognise that the fungal pathogens and diseases, and bacterial pathogens and diseases disclosed herein are exemplary only, and the scope of the present disclosure is not limited thereto. Numerous other fungal pathogens and diseases, and bacterial pathogens and diseases will be known to those skilled in the art, and methods and compositions of the present disclosure may also be used to combat these.

In accordance with the claimed invention, the composition comprises *Lactobacillus parafarraginis* Lp18 deposited with National Measurement Institute, Australia on 27 October 2011 under Accession Number V11/022945. In particular options disclosed herein but not necessarily forming part of the claimed invention, compositions disclosed herein comprise strains of one or more bacterial species selected from *Lactobacillus parafarraginis, Lactobacillus buchneri, Lactobacillus rapi, Lactobacillus zeae, Lactobacillus brevis* and *Lactobacillus diolivorans.* In alternate options, compositions may comprise a culture supernatant or cell free filtrate derived from culture media in which the above referenced strains have been cultured. In alternate options, compositions disclosed herein comprise strains of one or more bacterial species selected from *Lactobacillus parafarraginis, Lactobacillus buchneri, Lactobacillus rapi, Lactobacillus zeae, Lactobacillus casei. Lactobacillus paracasei,* strain designated herein 'T9' deposited with the National Measurement Institute, Australia on 14 December 2012 under Accession Number V12/022849, and strain designated herein 'TB' deposited with the National Measurement Institute, Australia on 14 December 2012 under Accession Number V12/022850.

The *Lactobacillus parafarraginis* strain in accordance with the claimed invention is *Lactobacillus parafarraginis* Lp18 deposited with National Measurement Institute, Australia on 27 October 2011 under Accession Number V11/022945. In other options disclosed herein but not forming part of the claimed invention, the *Lactobacillus buchneri* strain may be *Lactobacillus buchneri* Lb23. In a particular option the *Lactobacillus buchneri* strain is *Lactobacillus buchneri* Lb23 deposited with National Measurement Institute, Australia on 27 October 2011 under Accession Number V11/022946. The *Lactobacillus rapi* strain may be *Lactobacillus rapi* Lr24. In a particular option the *Lactobacillus rapi* strain is *Lactobacillus rapi* Lr24 deposited with National Measurement Institute, Australia on 27 October 2011 under Accession Number V11/022947. The *Lactobacillus zeae* strain may be *Lactobacillus zeae* Lz26. In a particular option the *Lactobacillus zeae* strain is *Lactobacillus zeae* Lz26 deposited with National Measurement Institute, Australia on 27 October 2011 under Accession Number V 11/022948.

In further options, compositions disclosed herein but not forming part of the claimed invention may comprise one or more strains selected from *Lactobacillus diolivorans* (N3) deposited with the National Measurement Institute, Australia on 14 December 2012 under Accession Number V12/022847, *Lactobacillus parafarraginis* (N11) deposited with the National Measurement Institute, Australia on 14 December 2012 under Accession Number V12/022848, *Lactobacillus brevis* (TD) deposited with the National Measurement Institute, Australia on 14 December 2012 under Accession Number V12/022851, *Lactobacillus paracasei,* strain designated herein 'T9' deposited with the National Measurement Institute, Australia on 14 December 2012 under Accession Number V12/022849, *Lactobacillus casei* and strain designated herein 'TB' deposited with the National Measurement Institute, Australia on 14 December 2012 under Accession Number V12/022850; or a culture supernatant or cell free filtrate derived from culture media in which one or more of these strains have been cultured.

Compositions of the present disclosure may further comprise a strain of *Acetobacter fabarum,* or a culture supernatant or cell free filtrate derived from culture media in which a strain of *Acetobacter fabarum* has been cultured. The *Acetobacter fabarum* strain may be *Acetobacter fabarum* Afl5. In a particular embodiment the *Acetobacter fabarum* strain is *Acetobacter fabarum* Afl5 deposited with the National Measurement Institute, Australia on 27 October 2011 under Accession Number V11/022943. Compositions may further comprise a yeast, or a culture supernatant or cell free filtrate derived from culture media in which a yeast has been cultured. The yeast may be a strain of *Candida ethanolica.* The *Candida ethanolica* strain may be *Candida ethanolica* Ce31. In a particular embodiment the *Candida ethanolica* strain is *Candida ethanolica* Ce31 deposited with the National Measurement Institute, Australia on 27 October 2011 under Accession Number V11/022944.

The concentrations of individual microbial strains to be added to compositions disclosed herein will depend on a variety of factors including the identity and number of individual strains employed, the microbial pathogen, infection or disease to be treated, the form in which a composition is applied and the means by which it is applied. For any given case, appropriate concentrations may be determined by one of ordinary skill in the art using only routine experimentation. By way of example only, the concentration of each strain present in the composition may be from about 1 × 10² cfu/ml to about 1 × 10¹⁰ cfu/ml. and may be about 1 × 10³ cfu/ml, about 2.5 × 10³ cfu/ml, about 5 × 10³ cfu/ml, 1 × 10⁴ cfu/ml, about 2.5 × 10⁴ cfu/ml, about 5 × 10⁴ cfu/ml, 1 × 10⁵ cfulml, about 2.5 × 10⁵ cfulml, about 5 × 10⁵ cfu/ml, 1 × 10⁶ cfu/ml, about 2.5 × 10⁶ cfu/ml, about 5 × 10⁶ cfu/ml, 1 × 10⁷ cfulml, about 2.5 × 10⁷ cfu/ml, about 5 × 10⁷ cfu/ml, 1 × 10⁸ cfu/ml, about 2.5 × 10⁸ cfulml, about 5 × 10⁸ cfulml, 1 × 10⁹ cfulml, about 2.5 × 10⁹ cfu/ml, or about 5 × 10⁹ cfu/ml. In particular exemplary embodiments the final concentration of the Lactobacillus strains is about 2.5 × 10⁵ cfu/ml. the final concentration of *Acetobacter fabarum* may be about 1 × 10⁶ cfu/ml and the final concentration of *Candida ethanolica* may be about 1 × 10⁵ cfu/ml.

Also contemplated by the present disclosure but not forming part of the invention are variants of the microbial strains described herein. As used herein, the term "variant" refers to both naturally occurring and specifically developed variants or mutants of the microbial strains disclosed and exemplified herein. Variants may or may not have the same identifying biological characteristics of the specific strains exemplified herein, provided they share similar advantageous properties in terms of treating or preventing infections caused by, or treating or preventing diseases caused by or associated with, microbial pathogens. Illustrative examples of suitable methods for preparing variants of the microbial strains exemplified herein include, but are not limited to. gene integration techniques such as those mediated by insertional elements or transposons or by homologous recombination, other recombinant DNA techniques for modifying, inserting, deleting, activating or silencing genes, intraspecific protoplast fusion, mutagenesis by irradiation with ultraviolet light or X-rays, or by treatment with a chemical mutagen such as nitrosoguanidine, methyl methane sulfonate, nitrogen mustard and the like. and bacteriophage-mediated transduction. Suitable and applicable methods are well known in the art and are described, for example, in J. H. Miller. Experiments in Molecular Genetics, Cold Spring Harbor Laboratory Press, Cold Spring Harbor, N.Y. (1972); J. H. Miller. A Short Course in Bacterial Genetics, Cold Spring Harbor Laboratory Press, Cold Spring Harbor, N.Y. (1992); and J. Sambrook, D. Russell, Molecular Cloning: A Laboratory Manual, 3rd ed., Cold Spring Harbor Laboratory Press, Cold Spring Harbor, N.Y. (2001). *inter alia.*

Also encompassed by the term "variant" as used herein are microbial strains phylogenetically closely related to strains disclosed herein and strains possessing substantial sequence identity with the strains disclosed herein at one or more phylogenetically informative markers such as rRNA genes, elongation and initiation factor genes, RNA polymerase subunit genes, DNA gyrase genes, heat shock protein genes and *recA* genes. For example, the 16S rRNA genes of a "variant" strain as contemplated herein may share about 85%, 86%, 87%, 88%, 89%, 90%, 91%, 92%, 93%, 94%, 95%, 96%, 97%, 98% or 99% sequence identity with a strain disclosed herein.

Methods of the present disclosure may further comprise administering to a subject in need of treatment, or otherwise exposing the subject to, one or more antimicrobial agents. Administration or exposure to a composition disclosed herein and an antimicrobial agent may be at the same time or at different times, *i.e*. simultaneous or sequential. Antimicrobial agents may be co-formulated with microbial strains used in the methods. Compositions disclosed herein may therefore comprise one or more antimicrobial agents. In instances where the microbial strains and antimicrobial agents are formulated in different compositions, they can be administered or delivered by the same or different routes or means.

Exemplary antimicrobial agents suitable for the methods described herein include, but are not limited to, antibiotics, detergents, surfactants, agents that induce oxidative stress, bacteriocins and antimicrobial enzymes (*e.g*. lipases, pronases and lyases) and various other proteolytic enzymes and nucleases, peptides and phage. The antimicrobial agents may be natural or synthetic. The antimicrobial agent employed may be selected for the particular application of the invention on a case-by-case basis, and those skilled in the art will appreciate that the scope of the present invention is not limited by the nature or identity of the particular antimicrobial agent. Non-limiting examples of antimicrobial agents include fluoroquinolones, aminoglycosides, glycopeptides, lincosamides, cephalosporins and related beta-lactams, macrolides, nitroimidazoles, penicillins, polymyxins, tetracyclines, and any combination thereof. For example, the methods of the present invention can employ acedapsone; acctosulfone sodium; alamecin; alexidine; amdinocillin; amdinocillin pivoxil; amicyclinc; amifloxacin; amifloxacin mesylate; amikacin; amikacin sulfate; aminosalicylic acid; aminosalicylate sodium; amoxicillin; amphomycin; ampicillin; ampicillin sodium; apalcillin sodium; apramycin; aspartocin; astromicin sulfate; avilamycin; avoparcin; azithromycin; aziocillin; azlocillin sodium; bacampicillin hydrochloride; bacitracin; bacitracin methylene disalicylatc; bacitracin zinc; bamhermycins; benzoylpas calcium; berythromycin; betamicin sulfate; biapenem; biniramycin; biphenamine hydrochloride; bispyrithione magsulfex; butikacin; butirosin sulfate: capreomycin sulfate; carbadox; carbenicillin disodium; carbenicillin indanyl sodium; carbenicillin phenyl sodium; carbenicillin potassium; carumonam sodium; cefaclor; cefadroxil; cefamandole; cefamandole nafate; cefamandole sodium; cefaparole; cefatrizine; cefazaflur sodium; cefazolin; cefazolin sodium: cefbuperazone; cefdinir; cefepime; cefepime hydrochloride; cefetecol; cefixime; cefmenoxime hydrochloride; cefmetazole; cermetazole sodium; cefonicid monosodium: cefonicid sodium; cefoperazone sodium; ceforanide; cefotaxime sodium; cefotetan; cefotetan disodium; cefotiam hydrochloride; cefoxitin; cefoxitin sodium: cefpimizole; cefpimizole sodium; cefpiramide; cefpiramide sodium; cefpirome sulfate; cefpodoxime proxctil; cefprozil; cefroxadine; cefsulcxiin sodium; ceftazidime; ceftibuten; ceftizoxime sodium; ceftriaxone sodium; cefuroxime: cefuroxime axetil; cefuroxime pivoxetil; cefuroxime sodium; cephacetrile sodium; cephalexin; cephalexin hydrochloride; cephaloglycin; cephaloridine; cephalothin sodium; cephapirin sodium; cephradine; cetocycline hydrochloride; cetophenicol; chloramphenicol; chloramphenicol palmitate; chloramphenicol pantothenate complex; chloramphenicol sodium succinate; chlorhexidine phosphanilate; chloroxylenol; chlortetracycline bisulfate; chlortetracycline hydrochloride; cinoxacin; ciprofloxacin; ciprofloxacin hydrochloride; cirolcmycin; clarithromycin; clinailoxacin hydrochloride; clindamycin; clindamycin hydrochloride; clindamycin palmitate hydrochloride; clindamycin phosphate; clofazimine; cloxacillin benzathine; cloxacillin sodium; chlorhexidine. cloxyquin; colistimethate sodium; colistin sulfate; coumermycin; coumermycin sodium; cyclacillin; cycloserine; dalfopristin; dapsone; daptomycin; demcclocycline; demcclocycline hydrochloride; demecycline; denofungin; diaveridine; dicloxacillin; dicloxacillin sodium; dihydrostreptomycin sulfate; dipyrithione; dirithromycin; doxycycline; doxycycline calcium; doxycycline fosfatex; doxycycline hyclate; droxacin sodium; enoxacin; epicillin; epitetracycline hydrochloride; erythromycin; erythromycin acistrate; erythromycin estolate; erythromycin ethyl succinate; erythromycin gluceptate; erythromycin lactobionate; erythromycin propionate; erythromycin stearate; ethambutol hydrochloride; ethionamide; fleroxacin; floxacillin; fludalanine; flumequine; fosfomycin; fosfomycin tromethamine; fumoxicillin; furazolium chloride; furazolium tartrate; fusidate sodium; fusidic acid; ganciclovir and ganciclovir sodium; gentamicin sulfate; gloximonam; gramicidin; haloprogin; hetacillin; hctacillin potassium; hexedine; ibafloxacin; imipenem; isoconazole; isepamicin; isoniazid; josamycin; kanamycin sulfate; kitasamycin; levofuraltadone; levopropylcillin potassium; lexithromycin; lincomycin; lincomycin hydrochloride; lomefloxacin; lomefloxacin hydrochloride; lomefloxacin mesylate; loracarbef; mafenide; mcclocycline; mcclocycline sulfosalicylate; megalomicin potassium phosphate: mequidox; meropenem; methacycline; methacycline hydrochloride; methenamine; methenamine hippurate; methenamine mandelate; methicillin sodium; metioprim; metronidazole hydrochloride; metronidazole phosphate; mezlocillin; mezlocillin sodium; minocycline; minocycline hydrochloride; mirincamycin hydrochloride; monensin: monensin sodiumr; nafcillin sodium; nalidixate sodium; nalidixic acid; natainycin; nebramycin; neomycin palmitate; neomycin sulfate; neomycin undecylenate; netilmicin sulfate; neutramycin; nifuiradene; nifuraldezone; nifuralel; nifuratronc; nifurdazil; nifurimide; nifiupirinol; nifurquinazol; nifurthiazole; nitrcxycline: nitrofurantoin; nitromide; norfloxacin; novobiocin sodium; ofloxacin; onnetoprim, oxacillin and oxacillin sodium; oximonam; oximonam sodium; oxolinic acid; oxytetracycline; oxytetracycline calcium; oxytetracycline hydrochloride; paldimycin; parachlorophenol; paulomycin: pefloxacin; pefloxacin mesylate; penamecillin; penicillins such as penicillin G benzathine, penicillin G potassium, penicillin G procaine, penicillin G sodium, penicillin V, penicillin V benzathine, penicillin V hydrabamine. and penicillin V potassium; pcntizidone sodium; phenyl aminosalicylate; piperacillin sodium; pirbenicillin sodium; piridicillin sodium; pirlimycin hydrochloride; pivampicillin hydrochloride; pivampicillin pamoate; pivampicillin probenate; polymyxin b sulfate; porfiromycin; propikacin; pyrazinamide; pyrithione zinc; quindecamine acetate; quinupristin; raccphcnicol; ramoplanin; ranimycin; relomycin; repromicin; rifabutin; rifametane; rifamexil; rifamide; rifampin; rifapentine; rifaximin; rolitelracycline; rolitetracycline nitrate: rosaramicin; rosaramicin butyrate; rosaramicin propionate; rosaramicin sodium phosphate; rosaramicin stearate; rosoxacin; roxarsone; roxithromycin; sancycline; sanfetrinem sodium; sarmoxicillin; sarpicillin; scopafungin; sisomicin; sisomicin sulfate; sparfloxacin; spectinomycin hydrochloride; spiramycin; stallimycin hydrochloride; steffimycin; streptomycin sulfate; streptonicozid; sulfabenz; sulfabenzamide; sulfacetamide; sulfacetamide sodium; sulfacytine; sulfadiazine; sulfadiazine sodium; sulfadoxine; sulfalene; sulfamerazine; sulfameter; sulfamethazine; sulfamethizole; sulfamethoxazole; sulfamonomethoxine; sulfamoxole; sulfanilate zinc; sulfanitran; sulfasalazine: sulfasomizole: sulfathiazole; sulfazamet; sulfisoxazole; sulfisoxazole acetyl; sulfisboxazole diolamine: sulfomyxin; sulopenem; sultamricillin; suncillin sodium; talampicillin hydrochloride; teicoplanin; temafloxacin hydrochloride; temocillin; tetracycline; tetracycline hydrochloride; tetracycline phosphate complex; tetroxoprim; thiamphenicol; thiphencillin potassium; ticarcillin cresyl sodium; ticarcillin disodium; ticarcillin monosodium; ticlatone; tiodonium chloride; tobramycin; tobramycin sulfate; tosufloxacin; trimethoprim; trimethoprim sulfate; trisulfapyrimidines; troleandomycin; trospectomycin sulfate; tyrothricin; vancomycin; vancomycin hydrochloride; virginiamycin; zorbamycin; and combinations thereof.

Compositions disclosed herein may optionally further comprise one or more additional microbial organisms, for example, agronomically beneficial microorganisms. Such agronomically beneficial microorganisms may act in synergy or concert with, or otherwise cooperate with the organisms of the present disclosure. Examples of agronomically beneficial microorganisms include *Bacillus* sp., *Pseudomonas* sp., *Rhizobium* sp., *Azospirillum* sp.. *Azotobacter* sp., phototrophic and cellulose degrading bacteria. *Clostridium* sp., *Trichoderma* sp. and the like. Those skilled in the art will appreciate that this list is merely exemplary only, and is not limited by reference to the specific examples here provided.

In the soil environment, inoculated bacteria can find survival difficult among naturally occurring competitor and predator organisms. To aid in survival of microorganisms present in compositions of the present disclosure upon application in the environment, one or more of the strains may be encapsulated in, for example, a suitable polymeric matrix. In one example, encapsulation may comprise alginate beads such as has been described by Young et al, 2006, Encapsulation of plant growth-promoting bacteria in alginate beads enriched with humic acid. Biotechnology and Bioengineering 95:76-83 . Those skilled in the art will appreciate that any suitable encapsulation material or matrix may be used. Encapsulation may be achieved using methods and techniques known to those skilled in the art. Encapsulated microorganisms can include nutrients or other components of the composition in addition to the microorganisms.

Compositions disclosed herein may be applied or administered directly or indirectly to any plant or animal in need of treatment. In the case of application to plants. compositions may be applied to plant parts (such as foliage) or seeds, or alternatively may be applied to soil in which the plants are growing or to be grown or in which seeds have been or are to be sown. Application may be by any suitable means and may be on any suitable scale. For example, application may comprise pouring, spreading or spraying, including broad scale or bulk spreading or spraying, soaking of seeds before planting, and/or drenching of seeds after planting or seedlings. Those skilled in the art will appreciate that multiple means of application may be used in combination (for example soaking of seeds prior to planting followed by drenching of planted seeds and/or application to seedlings or mature plants). Seeds, seedlings or mature plants may be treated as many times as appropriate. The number of applications required can readily be determined by those skilled in the art depending on, for example, the plant in question, pathogen or disease to be treated, the stage of development of the plant at which treatment is initiated, the state of health of the plant, the growth, environmental and/or climatic conditions in which the plant is grown and the purpose for which the plant is grown.

Thus, in accordance with the present disclosure, compositions disclosed herein may be prepared in any suitable form depending on the means by which the composition is to be applied to the soil or to plant seeds or vegetation. Suitable forms can include, for example, slurries, liquids, and solid forms. Solid forms include powders, granules, larger particulate forms and pellets. Solid forms can be encapsulated in water soluble coatings (for example dyed or undyed gelatin spheres or capsules), extended release coatings, or by micro-encapsulation to a free flowing powder using one or more of. for example, gelatin, polyvinyl alcohol, ethylcellulose, cellulose acetate phthalate, or styrene maleic anhydride. Liquids may include aqueous solutions and aqueous suspensions, and emulsifiable concentrates.

In order to achieve effective dispersion, adhesion and/or conservation or stability within the environment of compositions disclosed herein, it may be advantageous to formulate the compositions with suitable carrier components that aid dispersion, adhesion and conservation/stability. Suitable carriers will be known to those skilled in the art and include, for example, chitosan, vermiculite, compost, talc, milk powder, gels and the like.

Additional components may be incorporated into compositions of the present disclosure, such as humic substances, trace elements, organic material, penetrants, macronutrients, micronutrients and other soil and/or plant additives.

Humus or humic substances that may be incorporated may include, but are not limited to, humic acid derived from, for example oxidised lignite or leonardite, fulvic acid and humates such as potassium humate.

Organic material added may include, but is not limited to. biosolids, animal manure, compost or composted organic byproducts, activated sludge or processed animal or vegetable byproducts (including blood meal, feather meal, cottonseed meal. ocean kelp meal, seaweed extract, fish emulsions and fish meal).

Penetrants include, but are not limited to. non-ionic wetting agents, detergent based surfactants, silicones, and/or organo-silicones. Suitable penetrants will be known to those skilled in the art, non-limiting examples including polymeric polyoxyalkylenes, allinol, nonoxynol, octoxynol, oxycastrol, TRITON, TWEEN, Sylgard 309, Silwet L-77, and Herbex (silicone/surfactant blend).

Exemplary trace elements for inclusion in compositions are provided in Example 3. However those skilled in the art will recognise that suitable trace elements are not limited thereto, and that any trace elements (natural or synthetic) may be employed.

Optional further soil and/or plant additives that can be added to compositions of the present disclosure include, for example, water trapping agents such as zeolites, enzymes, plant growth hormones such as gibberellins, and pest control agents such as acaracides, insecticides, fungicides and nematocides.

Compositions of the present disclosure, including compositions comprising encapsulated strains may be freeze dried to extend shelf life and/or to aid in agricultural applications such as field dispersal.

The present disclosure will now he described with reference to the following specific examples, which should not be construed as in any way limiting the scope of the invention.

### Examples

Only examples comprising *Lactobacillus parafarraginis* strain Lp18 are illustrative of the invention. The examples should not be construed as limiting in any way the general nature of the disclosure of the description throughout this specification.

### Example 1- Microbial strains and strain combinations

The following microbial strains and strain combinations were used in the experiments described herein.

*Lactobacillus parafarraginis* Lp 18 was isolated from an environmental source. Partial 16S rRNA sequencing indicated 100% similarity to *Lactobacillus parqfarraginis* AB 262735 which has a risk group of 1 (TRBA). When cultured on MRS media for 3 days at 34°C, anaerobically, Lp 18 produces cream, round, slight sheen, convex, colony diameter 1-2mm (frlcultative anaerobe). Its microscopic appearance is Gram positive, non-motile, short rods rectangular, mainly diploid. *Lactobacillus parafarraginis* Lp 18 was deposited with the National Measurement Institute, Australia on 27 October 2011 under Accession Number V 11/022945.

*Lactobacillus buchneri* Lb23 was isolated from an environmental source. Partial 16S rRNA sequencing indicated 99% similarity to *Lactobacillus buchneri* AB 429368 which has a risk group of 1 **(TRBA).** When cultured on MRS media for 4 days at 34°C, anaerobically, Lb23 produces cream, shiny, convex., colony diameter 1-2rnm (facultative anaerobe). Its microscopic appearance is Gram positive, non-motile, rods in chains. *Lactobacillus buchneri* Lb23 was deposited with the National Measurement Institute, Australia 011 27 October 2011 under Accession Number Vl1/022946.

*Lactobacillus rapi* Lr24 was isolated from an environmental source. Partial 16S rRNA sequencing indicated 99% similarity to *Lactobacillus rapi* AB 366389 which has a risk group of 1 (DSMZ). When cultured on MRS media for 4 days at 34°C, anaerobically, Lr24 produces cream, round, shiny colonies with a diameter of 0.5mm (facultative anaerobe). Its microscopic appearance is Gram positive, non-motile, short rods single or diploid. *Lactobacillus rapi* Lr24 was deposited with the National Measurement Institute, Australia on 27 October 2011 under Accession Number V11/022947.

The fermentation results of API^{®} 50 CH strips (Biomerieux) show Lr24 only ferments L-Arabinosc. D-Ribose, D-Xylose, D-Fructose and Esculin ferric citrate.

*Lactobacillus zeae* Lz26 was isolated from an environmental source. Partial 16S rRNA sequencing indicated 99% similarity to *Lactobacillus zeae* AB 008213.1 which has a risk group of 1 (TRBA). When cultured on MRS media for 48 hours at 34°C, anaerobically. Lz26 produces white, round, shiny, convex, colonies with a diameter of 1mm (facultative anaerobe). Its microscopic appearance is Gram positive, non-motile, short rods almost coccoid, diploid and some chains. *Lactobacillus zeae* Lz26 was deposited with the National Measurement Institute, Australia on 27 October 2011 under Accession Number V11/022948.

The fermentation results of API^{®} 50 CH strips (Biomerieux) show Lz26 ferments D-Arabinose, D-ribose. D-Adonitol. D-Galactose, D-Glucose. D-Fructose, D-Mannose, L-Rhamnose, Dulcitol, Inositol, D-Mannitol, D-Sorbitol,N-Acetylglucosamine, Amygdalin. Arbutin, Esculin ferric citrate, Salicin, D-Cellobiose, D-Lactose, D-Trehalose, D-Melezitose, Gentiobiose, D-Turanose, D-Tagatose, L-fucose, L-Arabitol, Potassium gluconate and Potassium 2-Kctogluconate.

*Acetobacter fabarum* Af15 was isolated from an environmental source. Partial 16S rRNA sequencing indicated 100% similarity to *Acetobacter fabarum* AM 905849 which has a risk group of 1 (DSMZ). When cultured on Malt extract media for 3 days at 34°C, AF15 produces opaque, round, shiny, convex, colony diameter 1mm (aerobic). Its microscopic appearance is Gram negative, rods single or diploid. *Acetobacter fabarum* Af15 was deposited with the National Measurement Institute. Australia on 27 October 2011 under Accession Number V11/022943.

*Candida ethanolica* Cc31 was isolated from an environmental source. Partial 16S rRNA sequencing indicated 89% similarity to *Candida ethanolica* AB534618. When cultured on Malt extract media for 2 days at 34°C, Ce31 produces cream, flat, dull, roundish, colony diameter 2-3mm (aerobic). Its microscopic appearance is budding, ovoid yeast. *Candida ethanolica* Ce31 was deposited with the National Measurement Institute, Australia on 27 October 2011 under Accession Number V111022944.

Other strains used in experiments described herein were: *Lactobacillus diolivorans* (N3) deposited with the National Measurement Institute, Australia on 14 December 2012 under Accession Number V12/022847; *Lactobacillus parafarraginis* (N11) deposited with the National Measurement Institute, Australia on 14 December 2012 under Accession Number V12/022848; *Lactobacillus brevis* (TD) deposited with the National Measurement Institute, Australia on 14 December 2012 under Accession Number V12/022851; strain designated herein 'T9'. deposited with the National Measurement Institute, Australia on 14 December 2012 under Accession Number V121022849; and strain designated herein 'TB', deposited with the National Measurement Institute, Australia on 14 December 2012 under Accession Number V12/022850.

Strain designated herein 'TB', deposited with the National Measurement Institute, Australia on 14 December 2012 under Accession Number V12/U2285U, has a whole genome global similarity of 31.5% to *Lactobacillus casei,* as determined by a SILVA BLAST alignment search of small 16S ribosomal RNA (rRNA) sequences (Quast *et al.* 2013 The SILVA ribosomal RNA gene database project: improved data processing and web-based tools. *Nucl. Acid Res.* 41(D1):D590-596). The fermentation results of API^{®} 50 CH strips (Biomerieux) show TB ferments D-Galactose, D-Glucose. D-Fructose, D-Mannose, Methyl-aD-Mannopyranoside, Methyl-αD-Glucopyranoside, N-Acetylglucosamine. Amygdalin, Arbutin, Esculin ferric citrate, Salicin, D-Cellobiose, D-Maltose, D-Lactose, Sucrose, D-Melezitose, D-Raffinose, Gentiobiose, D-Tagatose and Potassium gluconate.

Strain designated herein T9'. deposited with the National Measurement Institute. Australia on 14 December 2012 under Accession Number V12/022849, has a whole genome global similarity of 95.3% to *Lactobacillus paracasei* as determined by a SILVA BLAST alignment search. The fermentation results of API^{®} 50 CH strips (Biomerieux) show T9ferments D-Ribose, D-Galactosc, D-Glucose, D-Fructose, D-Mannose, D-Mannitol, D-Sorbitol, Methyl-αD-Glucopyranoside, N-Acetylglucosamine, Amygdalin, Arbutin, Esculin ferric citrate. Salicin, D-Cellobiose. D-Maltose, Sucrose, D-Trehalose, Inulin, Gentiobiose, D-Turanose, D-Tagatose IfArabitol, Potassium gluconate and Potassium 2-Ketogluconate.

The following combinations of the above described microbial strains were used in the experiments described herein below.

The composition referred to herein below as the 'GL composition' comprises six microbial strains described above (namely *Acetobacter fabarum* Af15, *Lactobacillus parafarraginis* Lp18. *Lactobacillus buchneri* Lb23, *Lactobacillus rapi* Lr24. *Lactobacillus zeae* Lz26, and *Candida ethanolica* Ce31) at final concentrations of 2.5 × 10⁵ cfulml for each of the *Lactobacillus* strains, 1.0 × 10⁵ cfu/ml for *Candida ethanolica* Ce31 and 1.0 × 10⁶ cfu/ml for *Acetobacter fabarum*Af15*.*

The composition referred to herein below as 'Mix G' comprises three of the microbial strains described above, specifically *Lactobacillus zeae* Lz26. strain TB (NMI Accession Number V12/022850) and T9 (NMI Accession Number V12/022849). Fresh cultures of bacteria were added at the respective final concentrations 1 × 10⁷cfu/ml *Lactobacillus zeae* Lz26,1 × 10⁷cfu/ml TB and 1 × 10⁷cfu/ml T9, to a mix of 2% trace elements, 0.3% humic, 4% molasses and water. The composition was adjusted to pH 3.8-4.2 with phosphoric acid.

The composition referred to herein below as 'Mix Γ comprises five of the microbial strains described above, specifically *Lactobacillus zeae* Lz26, *Lactobacillus buchneri* Lb23, *Lactobacillus parafarraginis* Lp18. *Candida ethanolica* Ce31, and *Acetobacter fabarum* Af15. Fresh cultures of bacteria were added at the respective final concentrations 1 × 10⁷cfu/ml *Lactobacillus zeae* Lz26, 1 × 10⁷cfu/ml*Lactobacillus buchneri* Lb2.3,1 × 10⁷ cfu/ml *Lactobacillus parafarraginis* Lp18, 1 × 10⁵cfu/ml *Candida ethanolica* Ce31 and 1 × 10⁶cfu/ml *Acetobacter fabarum* Af15, to a mix of 2% trace elements, 0.3% humic, 4% molasses and water. The composition was adjusted to pH 3.8-4.2 with phosphoric acid.

The composition referred to herein below as 'Mix 2' comprises five of the microbial strains described above, specifically *Lactobacillus zeae* Lz26, *Lactobacillus parafarraginis* Lp18, *Lactobacillus buchneri* Lb23. *Lactobacillus rapi* Lr24, and *Acetobacter fabarum* Af15. Fresh cultures of bacteria were added at the respective final concentrations1 × 10⁷cfu/ml *Lactobacillus zeae* Lz26,1 × 10⁶cfu/ml *Lactobacillus parafarraginis* Lp18,1 × 10⁶cfu/ml *Lactobacillus buchneri* Lb23,1 × 10⁶cfu/ml *Lactobacillus rapi* Lr24 and1 × 10⁶cfu/ml *Acetobacter fabarum* Af15, to a sterile mix of 2% trace elements, 3% molasses and RO water.

The composition referred to herein below as 'Mix 3' comprises four of the microbial strains described above, specifically *Lactobacillus zeae* Lz26. *Lactobacillus parafarraginis* Lpl8, *Lactobacillus buchneri* Lb23 and *Lactobacillus rapi* Lr24. Fresh cultures of Lactobacillus sp. were added at the respective final concentrations 1 × 10⁷cfu/ml *Lactobacillus zeae* Lz26, 1 × 10⁶cfu/ml *Lactobacillus parafarraginis* Lp18, 1 × 10⁶cfu/ml *Lactobacillus buchneri* Lb23 and 1 × 10⁶cfu/ml *Lactobacillus rapi* Lr24, to a sterile mix of 3% molasses and RO water.

### Maintenance of cultures

30% glycerol stocks were made of each isolate and maintained at -80°C for long-term culture storage. Short-term storage of the cultures were maintained at 4°C on agar slopes (3 month storage) and on agar plates which are subcultured monthly. To maintain the isolates original traits, a fresh plate is made from the -80°C stock following three plate subcultures.

### Inoculum and growth media

The *Lactobacillus* strains were grown with or without air (*L. rapi* prefers anaerobic) either in MRS broth (Difco) or on MRS agar plates depending on application. The cultures were routinely grown for 2 days at a mesophilic temperature of 30-34°C. The *Acetobacter* and *Ethanolica* strains were grown aerobically either in Malt extract broth (Oxoid) or on Malt extract agar plates depending on application. The cultures were routinely grown for 2 days at a mesophilic temperature of 30-34°C.

### Fermenter 'seed' preparation

For individual strains, using a sterile nichrome wire a single colony is removed from a fresh culture plate and transferred to a universal bottle containing 15mL of sterile media. The bottle is securely placed in a shaking incubator set at 30°C, 140rpm for 48hrs (*L. rapi* is not shaken). After incubation a cloudy bacterial growth should be visible. 'Seed' inoculation bottles are stored at 4°C until required (maximum 1 week).

Typically a 5% bacterial inoculation is required for a fermentation run. The stored 15ml culture seed is added to a Schott bottle containing a volume of sterile media which is 5% of the total fermenter working volume. The culture is incubated and shaken in the same way as the 15ml seed. Large-scale automatic fermenters are used to grow pure cultures of each isolate. There is an automatic feed of alkali, antifoam and glucose. Typically the temperature is maintained at 30-34°C, pH 5.5 but the oxygen and agitation varies depending on the microorganism.

### Sample analysis

After each large scale culturing of an isolate a sample is aseptically withdrawn and a viability count undertaken using 10 fold serial dilutions, performed in a laminar flow hood. A wet slide is also prepared and purity observed using a phase contrast microscope to double check for contaminants that may be present but unable to grow on the culture media. After 48 hours the viability plates are checked for a pure culture (same colony morphology) and the colonies counted to produce a colony forming unit per ml (cfu/ml) value. A Grams stain was also performed for microscopic observation.

### Example 2 - Activity against Fusarium oxysporum f.sp zingiberi

Ginger yellows was first reported in Queensland in 1955. It is caused by ***Fusarium** oxysporum* f. sp *zingiberi* which causes yellowing and wilting of the leaves and rhizome rot of the ginger. This disease has caused serious economic loss in Australia's ginger industry.

The inventors conducted a laboratory experiment in which the plant pathogen *Fusarium oxysporum* f. sp *zingiberi* was challenged with individual bacterial strains described in Example 1 (hereinafter "GL" strains) to determine if they show an antagonistic effect against the *Fusarium* ginger pathogen.

A pure isolate of *Fusarium oxysporum* f. sp *zingiberi* (hereinafter *"foz"*) was purchased from the Herbarium (BRIP) Queensland DPI culture collection. *Foz* was routinely grown on PDA solid media aerobically at room temperature. After a few days a pink growth is seen and after around 5 days white aerial mycelium develop. It also grows well on malt extract (ME) and MRS solid media. *Lactobacillus parafarraginis* Lp18, *Lactobacillus buchneri* Lb23. *Lactobacillus rapi* Lr24. and *Lactobacillus zeae* Lz26 were routinely grown anaerobically on MRS media at 34°C. *Acetobacter fabarum* Af15 and *Candida ethanolica* Ce31 were routinely grown aerobically on ME agar at 34°C. The antifungal activity of the GL bacterial strains was determined using four different methods, well plates, cross streak plates, a dual plate screen and a culture drop method according to the following experimental protocols.

### Well plates

- Two agar plates were prepared specific to each pathogens growth requirements.
- Using the large end of a 1ml sterile tip, six 9mm holes were cut into one plate and seven 9mm holes cut in the second plate.
- 2.5mls of sterile 0.85% saline was added to a sterile bijou bottle. Using a flamed loop 2-3 colonies (or 2 loops of fungal hyphal growth) were removed from a freshly grown pathogen plate, washed off into the saline and vortexed well such that the solution looked similar to a 0.5 MacFarlands standard.

- A sterile swab was dipped into the saline/colony mix and using gentle pressure, the swab was zigzaged across the plate, careful not to destroy the wells. This was repeated three times turning the plate slightly each time.
- A freshly grown broth (1-3 days) of the specific GL strain to be tested, was filtered through a sterile 0.45µm filter to remove the microbes. The filtrate was collected into a sterile bijou bottle.
- Each well of the plate was labelled with each of the GL strains to be tested. 80µl of the filtrate was added to each well.
- The plates were left, lid uppermost and incubated at room temperature.
- After 24 hours the plates were checked for pathogen growth. The diameters of any clear zones of inhibition (including the 9mm well diameter) were measured and recorded. If little or weak growth was seen the plates were left to incubate longer until good growth was seen.

### Cross streak plates

This method was used to investigate the effect of the growing GL strains *per se* on the pathogen rather than a supernatant filtrate.
- Using a flamed loop several vertical streaks (~1cm wide) of a fresh GL culture were made down the centre of an agar plate (relative to the bacterial growth requirements).
- The plates were incubated overnight at the relevant growth temperature to achieve a good growth of the GL strain isolate.
- Using a fresh plate culture of the pathogen, a single line was made horizontally across the plate from left to right. The plate was reincubated.
- After 24 hours of incubation the left hand side of the plates were observed for any growth inhibition areas between the fungal pathogen and GL strain inoculated lines (the right hand side is a mix of pathogen and GL strain). If necessary the plates were left incubating longer until a good growth of pathogen was seen.

### Dual Plate screen

This method was developed to circumvent the difficulties associated with different growth requirements of the GL strains and the pathogens. The screen was performed in a two section petri dish using two different growth media per section.
- Using sterile petri dishes with two sections the GL bacteria culture agar was poured in both sections.
- Once dried and set, half of each section was aseptically cut away. The removed quarter sections were filled with the pathogen growth media.
- 1-3 fresh GL strain colonies were added to 2.5 mls, sterile, 0.85% saline, to a similar density as a 0.5 McFarlands Standard.
- Using a sterile swab, three spreads were made per quarter section. The plates were incubated for 24-48hours at 34°C aerobically (or anaerobically), depending on the microbe.
- 3 sterile loop sized chunks of *foz* were cut from a fresh plate and washed in 5mls of sterile milli Q water and vortexed well to mix.
- Using a sterile swab, one spread was made across the pathogen agar careful to get close, but without touching the GL strain.
- The plates were double wrapped and incubated at the appropriate pathogen growth temperature (generally 27°C) for 1-10 days depending on the pathogens growth rate.
- The distance between the pathogen growth and GL strain edge were measured and recorded.

### Culture drop method

This was used to look at the effect of a viable culture of GL strains directly on the pathogen. A swabbed spread plate of *foz* was prepared as described above. 10µl of a freshly grown overnight culture of a GL strain was dropped on top of the *foz* spread plate in marked spots. The plates were left at room temperature overnight and observed after 24 and 48 hours growth. The clear inhibition zone diameters were recorded.

The results from the laboratory experiments are shown in Table 1. Actively growing *Lactobacillus parafarraginis* Lp18, *Lactobacillus buchneri* Lb23. *Lactobacillus rapi* Lr24 and *Lactobacillus zeae* Lz26 displayed the best growth inhibition of *foz.* The fact that cell free filtrates derived from growth cultures of these organisms did not inhibit *foz* growth suggests an interaction between the bacteria and the *Fusarium* pathogen. *Acetobacter fabarum* Af15 and *Candida ethanolica* Ce31 showed lesser ability to inhibit the growth of *foz*.

Also active against *foz* were actively growing strains *Lactobacillus diolivorans* (N3), *Lactobacillus brevis* (TD), and strains TB and T9, as well as cell free filtrates drawn from the culture of N3, TB, T9, TD and *Lactobacillus parafarraginis* (N11).

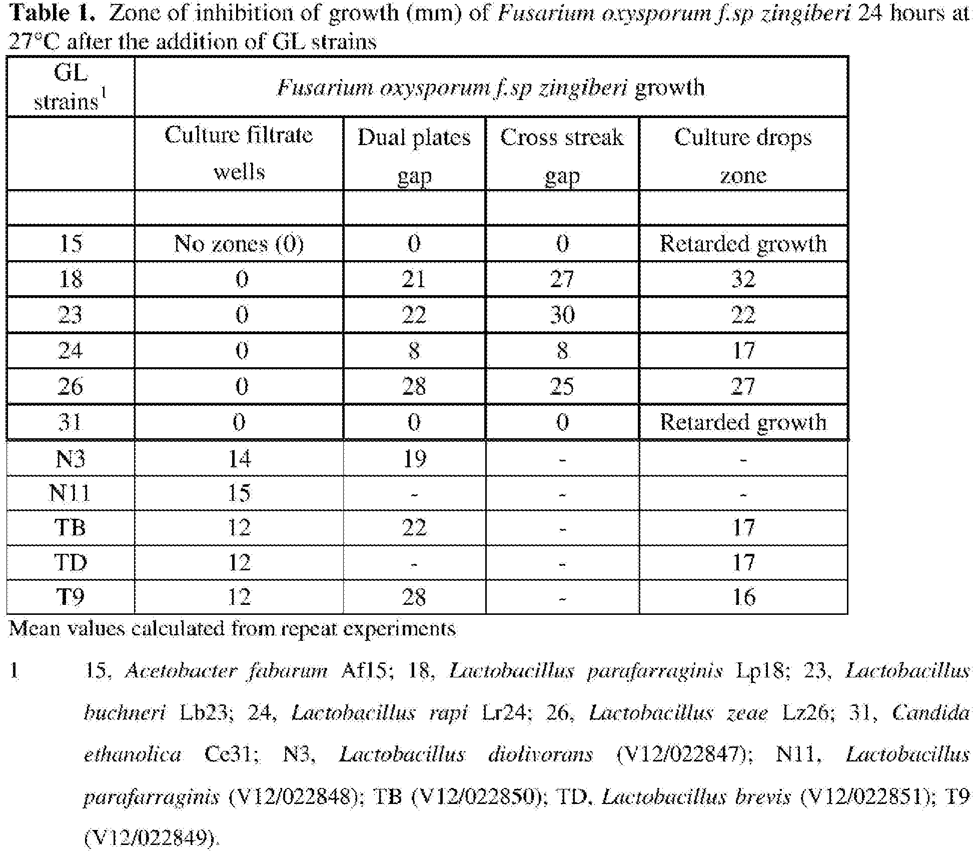

### Example 3 . Control of Fusarium in watermelon seedlings

### Infected seedlings

The inventors then investigated the effect of a composition comprising the bacterial strains described in Example 1 on the plant pathogen *Fusarium oxysporum* in Huntsman watermelon seedlings. *Fusarium oxysporum* has many specialized forms (f. sp). The form affecting watermelons causing Fusarium wilt is *Fusarium oxysporum* f. sp *niveum.* The seedlings used in this study were infected with *Fusarium oxysporum* and obtained from a watermelon farm with a significant *Fusarium* problem in the soil (provided by Jason Klotz).

The composition (referred to below as 'GL composition') comprised six microbial strains listed in Example 1. (namely *Acetobacter fabarum* Af15, *Lactobacillus parafarraginis* Lp18, *Lactobacillus buchneri* Lb23. *Lactobacillus rapi* Lr24. *Lactobacillus zeae* Lz26, and *Candida ethanolica* Ce31) at final concentrations of 2.5 × 10⁵ cfulml for each of the *Lactobacillus* strains, 1.0 × 10⁵ cfu/ml for *Candida ethanolica* and 1.0 × 10⁶ cfu/ml for *Acetobacter fabarum.* The strains were grown as described in Example 1 and mixed with 2% trace elements, 0.3% humate (Soluble Humate, LawrieCo), 3% molasses and 0.1-0.2% phosphoric acid. Phosphoric acid was added to the point where pH was in the range 3.8 to 4.0. The trace elements component typically comprised the following (per 1,000L):

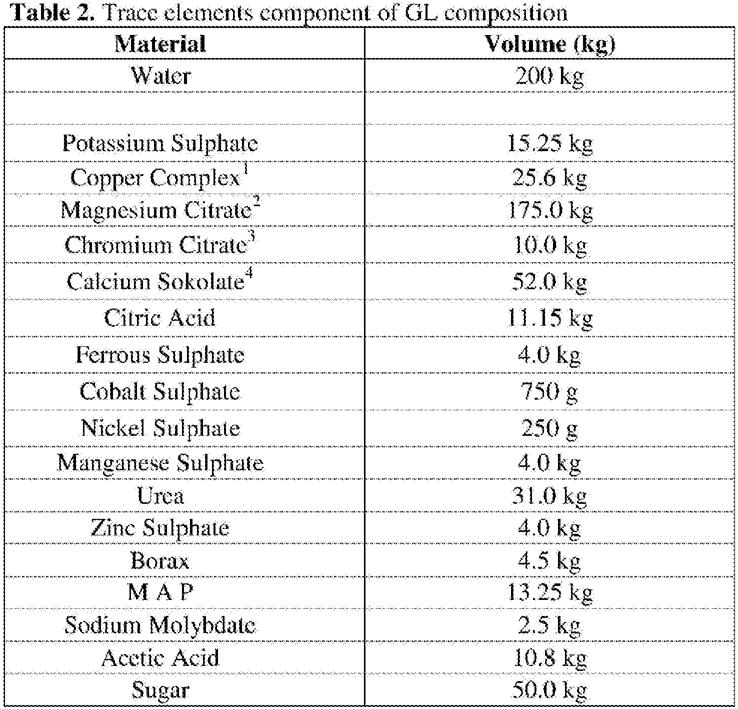

The experimental scheme is shown in Table 3. Each experiment had four replicates.

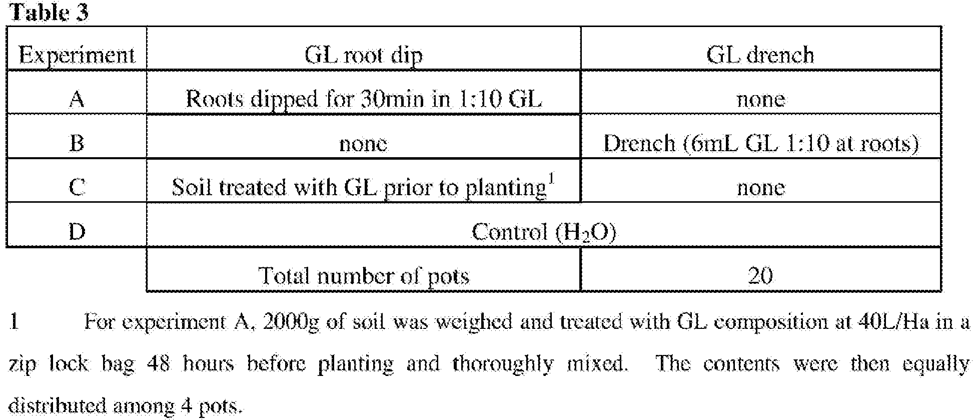

Huntsman melon seedlings known to be infected with *Fusarium oxysparum* were potted and placed under the hydroponic lights at ~28-30°C. Upon arrival the seedlings looked pale and yellow. The potted seedlings were watered and treated as described in Table 3. All pots were watered using a spray can twice a day. As the plants grew, the amount of water per plant was increased.

After initial observations one week after planting, all plants except those in the control experiment (D) were treated with 1:10 GL at 40L/Ha. The plants received a treatment once a week, for approximately three weeks.

Seven days after the initial treatment for experiments A, B and C it was observed that the plants were wilted and dry. 48 hours after the second treatment the plants in experiments A and B (and one pot in experiment C) appeared healthy. The plants in 3 pots in experiment C and the control remained dry and dead. Results are summarised below and in Figure 1.

Experiment A: 4/4 plants survived the *Fusarium oxysporum* infection. The plants had larger and more vibrant leaves in comparison to the other treatments. The plants had a much stronger main stem. The root system was significantly compact and dense in all replicates. The average weight of the plants was the highest and was a good indication of overall plant growth.

Experiment B: 3/4 plants survived the *Fusarium oxysporum* infection. One plant was affected by the pathogen after the first week. The leaf size was average and the plant health was good. The root system was less dense than in A. Overall plant health was satisfying. The main stem of the plants was strong.

Experiment C: 1/4 plants survived the *Fusarium oxysporum* infection. The overall plant growth and health was very poor.

Experiment D: All replicates were affected by the *Fusarium oxysporum* and died within a week.

In summary, the results indicate that soaking the roots of infected seedlings for 30min in 1:10 GL solution prior to planting was the most successful in comparison to other treatments and the H₂O control. It is clear that the introduction of the bacteria present in the GL solution to the rhizosphere of the roots increased the chances of plant survival against the plant pathogen *Fusarium oxysporum.*

### Protection of seedlings in infected soil

The inventors then investigated whether a microbial strain composition described herein can protect watermelon seedlings against *Fusarium* sp. infected soil.

### Experiment 1

Small propagation greenhouses containing 24 cell trays were used for the experiment. The trays were filled with a well mixed field soil known to be infected with a *Fusarium* species that attacks watermelons. Three seeds of 'Candy red' watermelon seeds were planted in each cell. There were three cells per formulation/control. Each trio was bagged underneath to prevent drainage run out cross contamination between formulations. A cell set contained a water control, an autoclaved soil control (3 autoclavings 2 days apart) and the microbial composition Mix G (see Example 1). Two cell sets were compared; soaked seeds and unsoaked seeds. Seeds were soaked for 1hour in either sterile water or Mix G (diluted 1 in 10). Each seed was planted to a similar depth and immediately after planting each cell was dosed with 6ml of sterile water or 1ml of Mix G (1:10) plus 5ml sterile water. The propagation houses were sealed and left at ambient temperature out of direct sunlight for 12 days.

### Experiment 2

Experiment 2 was essentially set up in the same way as Experiment 1 with two exceptions. Seeds were soaked for 30 mins and the initial microbial composition dose was reduced by a third (300µl of MixG diluted 1:10 was added to 3ml of sterile water) but repeated after 1 week. 3m1 sterile water was added to the water controls.

The results of Experiment 1 and Experiment 2 are shown in Table 4. Seeds soaked with Mix G, with a 7 day follow up second dose, successfully protected the watermelon seedlings against *Fusarium* infection over the 12 day growing period. Seed soak time affected the germination rate. The shorter soak time in Experiment 2 improved seedling height of all seedlings, compared to the comparable unsoaked seedlings. Interestingly, autoclaved soil devoid of all biological activity, retarded seedling growth in both experiments.

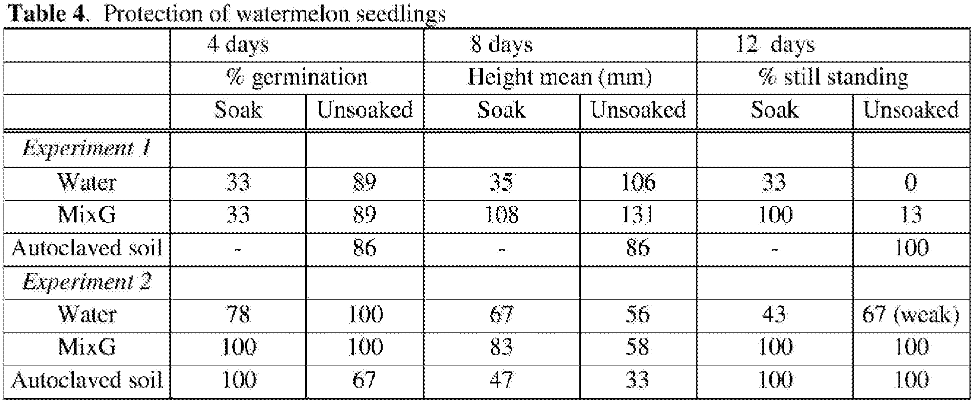

### Example 4 - Activity against Fusarium oxysporum f.sp cubense (Vegetative Compatabitity Group 0120) accession number 24322

*Fusarium oxysporum* f. sp *cubense (races 1-4)* is the causal pathogen of the destructive Panama disease in bananas. Due to strict quarantine containment the causal pathogen could not be directly tested in the laboratory, however, a less virulent strain was permitted (foc accession no. 24322). The dual plate assay was performed which challenged the pathogen to grow against the GL composition (see Example 3), and the individual GL strains described in Example 1. The experiment was performed in duplicate and repeated. The results are shown in Table 5. The results show that the GL composition completely inhibited *Fusarium* growth, and also show a strong antimicrobial effect from GL strains *Lactobacillus zeae*Lz26, *Lactobacillus brevis* (TD), TB and T9.

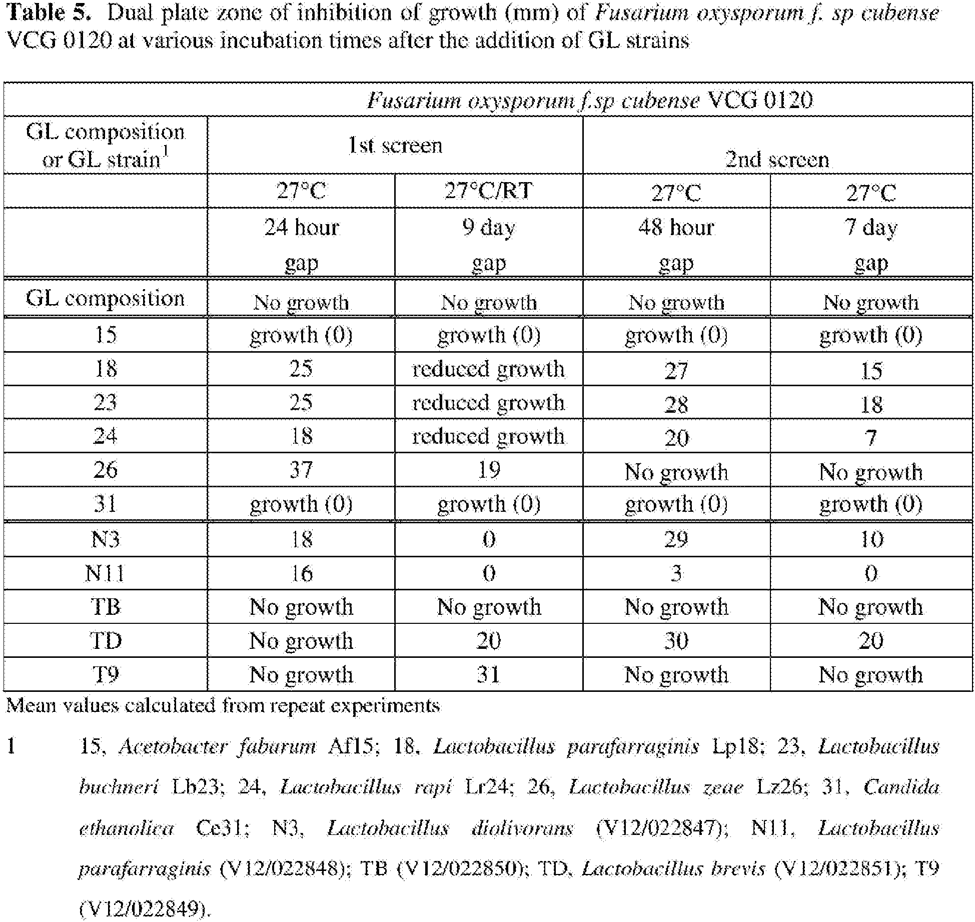

### Example 5 - Activity against other fungal species

*Pseudocercospora macadamiae* is the causal pathogen of husk spot of macadamia nuts. It causes the nuts to drop from the trees prematurely creating great economic loss to the macadamia industry. Some varieties of macadamia tree are more susceptible than others, such as A16. A pure isolate of *Pseudocercospora macadamiae* was acquired from the Queensland Department of Primary Industries culture collection. The ability of GL strains described in Example 1 to inhibit growth of this fungal pathogen was tested as described in Example 2. The results arc shown below in Table 6. *Lactobacillus parafarraginis* Lp18*.Lactobacillus buchneri* Lb23, *Lactobacillus rapi* Lr24. *Lactobacillus zeae* Lz26. *Lactobacillus diolivorans* (N3), *Lactobacillus brevis* (TD), TB and T9 were able to inhibit growth of the pathogen, using both actively growing culture and growth media (filtrate).

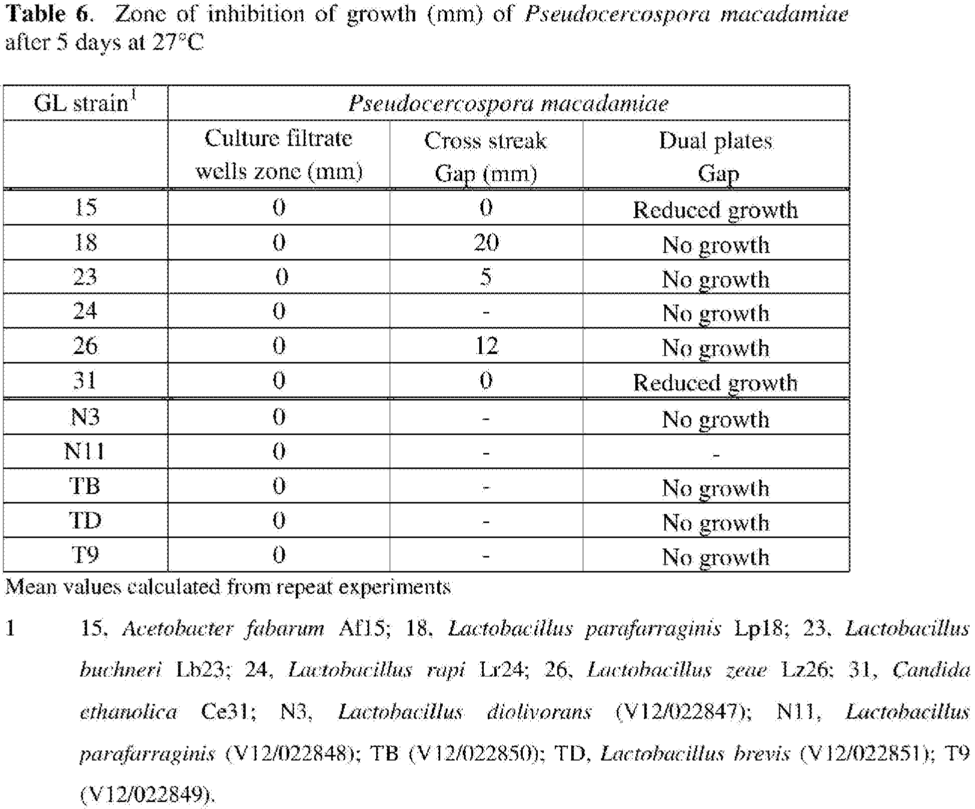

Similar experiments were carried out to determine the ability of GL strains described in Example 1 to inhibit growth of the fungal pathogens *Rhizoctonia solani* (causative agent of root rot, collar rot, damping off and wire stem in a range of plant species) and *Botrytis cinerea* (a nccrotrophic fungus affecting a wide variety of crops including grapes, tomatoes and strawberries). The dual plates screen method as described in Example 2 was used, with the exception that a small piece of fungal mat was directly placed at the top of the plate rather than being swabbed across the plate. The results are shown in Table 7.

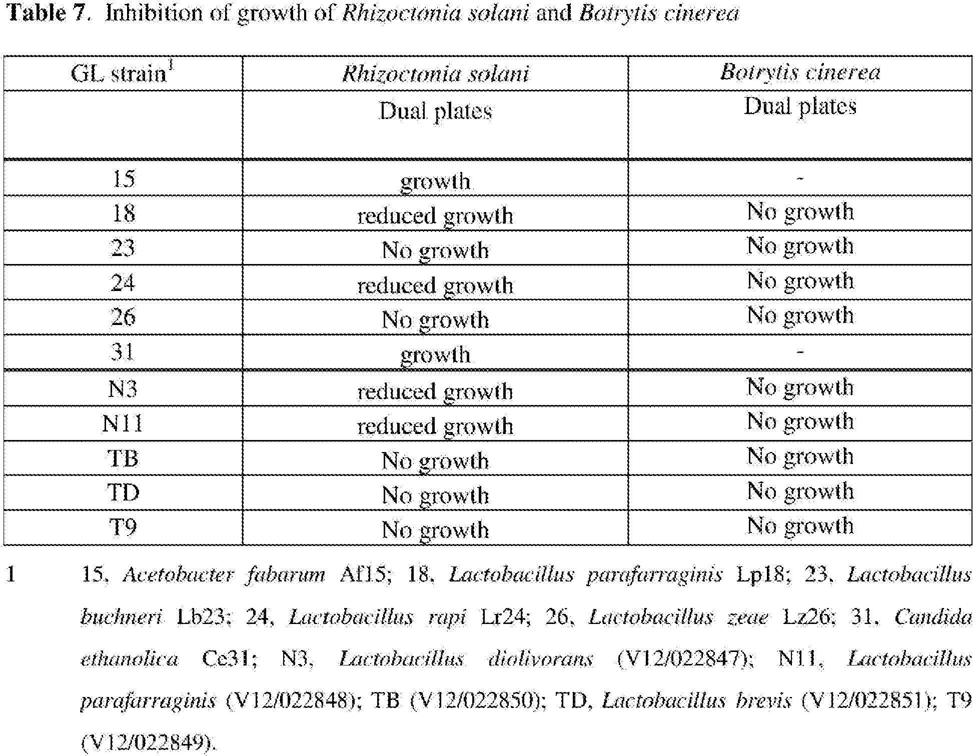

Similar experiments were carried out using the dual plates screen method to determine the ability of GL strains and mixes described in Example 1 to inhibit growth of the fungal pathogens *Alternaria solani, Colletrichum coccodes, Leptosphaeria maculans* and *Sclerotinia sclerotiorum.* Results are shown in Tables 8 and 9.

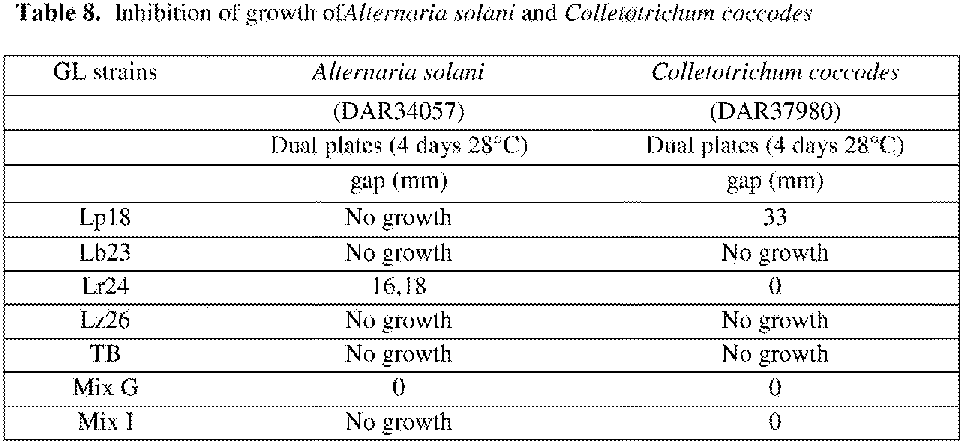

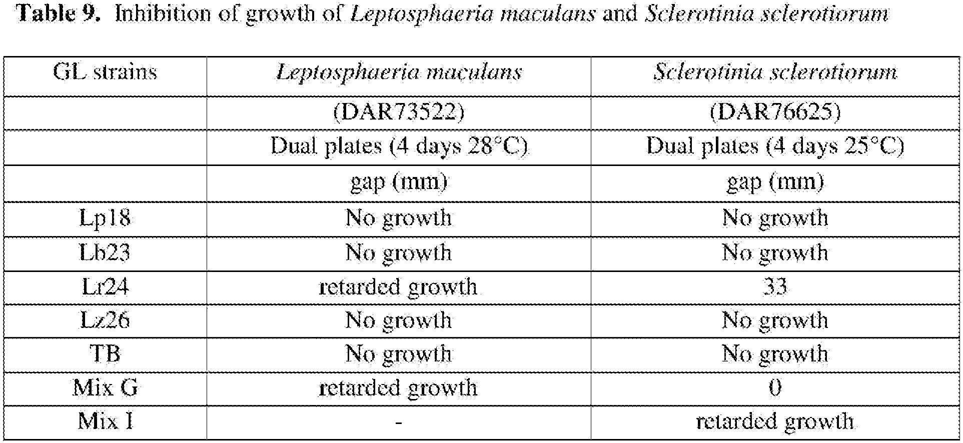

### Example 6- Activity against Streptomyces scabies

The inventors conducted a laboratory experiment to determine the ability of GL strains described in Example 1 to inhibit growth of the Gram positive bacterium *Streptomyces scabies,* the causative agent of potato scab.

A pure isolate of *Streptomyces scabies* was obtained from Anabel Wilson at The Vegetable Centre, Tasmanian Institute of Agriculture, New Town, Tasmania. *S. scabies* was routinely grown on PDA solid media aerobically at room temperature. After a few days a white growth is seen and after around 5 days grey aerial mycelium develop. *Lactobacilius parafarraginis* Lp18, *Lactobacillus buchneri* Lb23, *Lactobacillus rapi* Lr24, and *Lactobacillus zeae* Lz26 were routinely grown anaerobically on MRS media at 34°C. *Acetobacter fabarum* Af15 and *Candida ethanolica* Ce31 were routinely grown aerobically on ME agar at 34°C. The antibacterial activity of the bacterial strains was determined using three different methods. well plates, cross streak plates and a dual plate method as described in Example 2.

Results are shown below in Table 10. Actively growing *Lactobacillus parafarraginis* Lp18, *Lactobacillus buchneri* Lb23 and *Lactobacillus zeae* Lz26 and strain TB displayed best growth inhibition of *S. scabies.* The fact that cell free supernatant derived from cultures of these organisms did not inhibit *S*. *scabies* growth suggests an interaction between the GL strains and the pathogen.

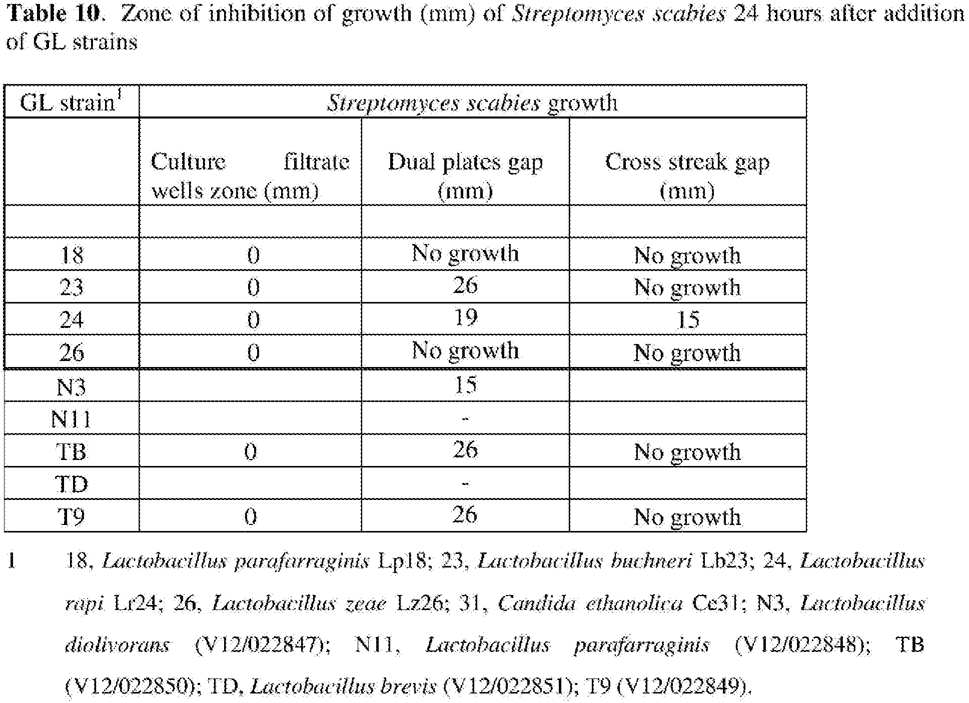

### Example 7- Activity against causative agents of mastitis

Clinical mastitis is a serious issue within the dairy industry. Mastitis can be caused by several different bacterial species, the principal causative species being the Grant-positive species *Staphylococcusaureus* and *Streptococcusuberis* and the Gram-negative species *Escherichia coli.* The inventors investigated the ability of GL strains described in Example 1 (alone and in combination) to inhibit growth of an environmental sample (isolate) of *Escherichia coli* , a laboratory strain of *Escherichia coli* (ATCC 25922) and laboratory strains of *Staphylococcus aureus* and *Streptococcus uberis.*

### Determination of MICs for bacterial culture filtrates

Fresh overnight cultures were grown of individual *Lactobacillus* GL strains. 3ml of the overnight culture was spun at 4,000rpm for 10mins. The supernatant was decanted and filtered through a 0.45µl syringe filter unit into a sterile bijoux bottle. The sterile filtrate from each bacterial culture as well as a control of MRS growth media was diluted 1:1, 1:3, 1:5, 1:10 using sterile 0.85% saline. Using the wide end of a sterile 1ml tip, six well spaced, holes were cut in a fresh nutrient agar plate. The plate was swabbed three times with one of the three mastitis pathogens (diluted to a 0.5 MacFarlands std). 80ul of each diluted filtrate (as well as undiluted and MRS) was added to each of the six wells. This was repeated for each pathogen and each bacterial filtrate. The plates were incubated overnight at 34°C. The diameter of clear zones of inhibited growth were measured and recorded.

Results are shown in Tables 11, 12 and 13. Growth culture filtrates from *Lactobacillus zeae* Lz26, TB and T9 demonstrated antimicrobial activity against all three mastitis pathogens. The filtrates remained effective up to a 1:3 dilution. All three pathogens grew well in the absence of GL strains.

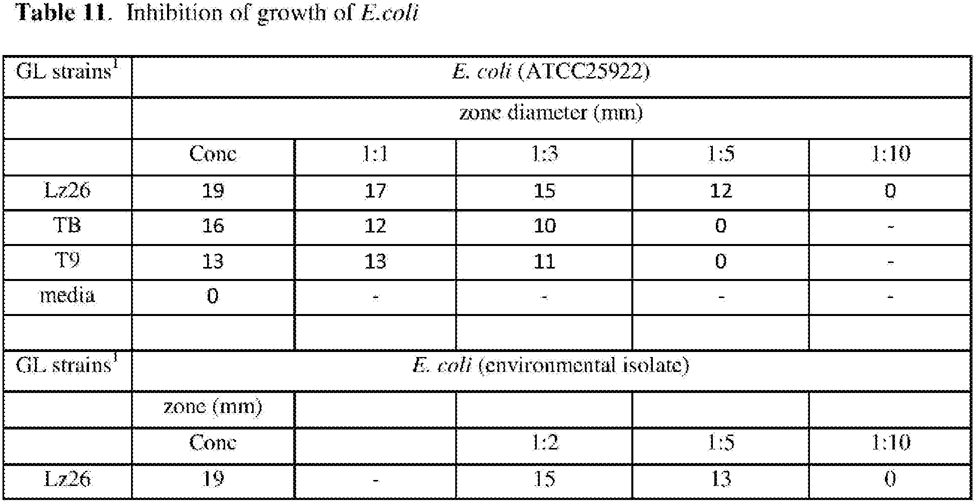

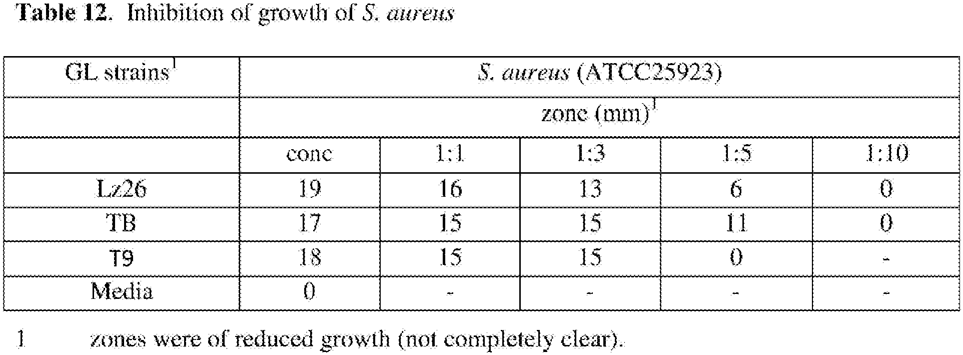

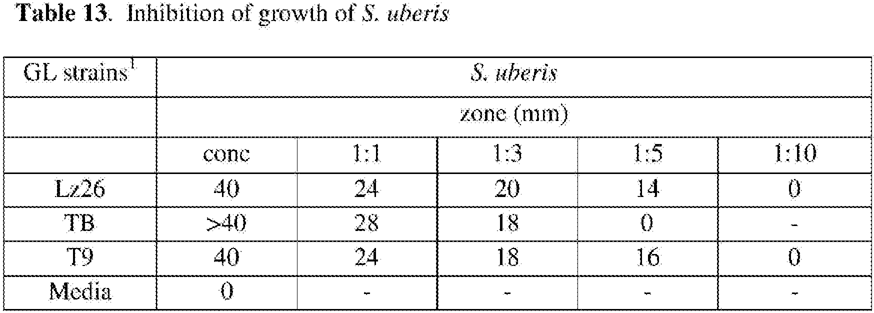

### Determination of minimum CFUs required to inhibit pathogens

The viability of each of six *Lactobacillus* GL strains and two compositions (Mix 2 and Mix 3; see Example 1) was determined at the start of the experiment. The number of colony forming units per ml (cfulml) of each culture was recorded (Table 14).

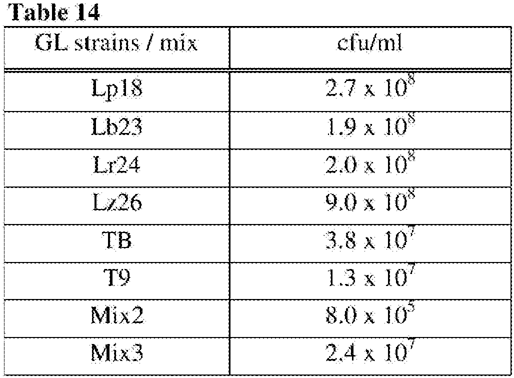

Each strain (or mix) was diluted in sterile MRS media 1:100, 1:1,000 and 1:10,000. Nutrient agar/MRS dual plates were poured (described above). 100µl of diluted culture was spread onto each MRS quarter of the dual plate (duplicates). A small glass 'hockey stick' was used to spread the 100µl over the media. The plates were incubated anaerobically at 34°C for 48 hours. The three pathogens were diluted to a 0.5 MacFarlands std and using a sterile swab each was swabbed across both nutrient agar quarters of the dual plate. The plates were reincubated, for 2 days at 34°C. Any clear zones of growth were measured and recorded. Zero indicated no zone (no inhibition) and no growth indicated no growth of the pathogen (complete inhibition). A swab line of each pathogen was drawn across a control nutrient agar plate to demonstrate their viability in the absence of the GL strain or composition.

Results are shown in Tables 15, 16 and 17. Viable cultures of T9 and Mix 3 were the most effective requiring less than 100 colonies to cause an inhibitory effect against all three mastitis causing bacterial species. Lactobacillus Lz26. Lb23 and T9 will be used to formulate a third anti-mastitis mix. All three pathogens grew well in the absence of GL strains/compositions.

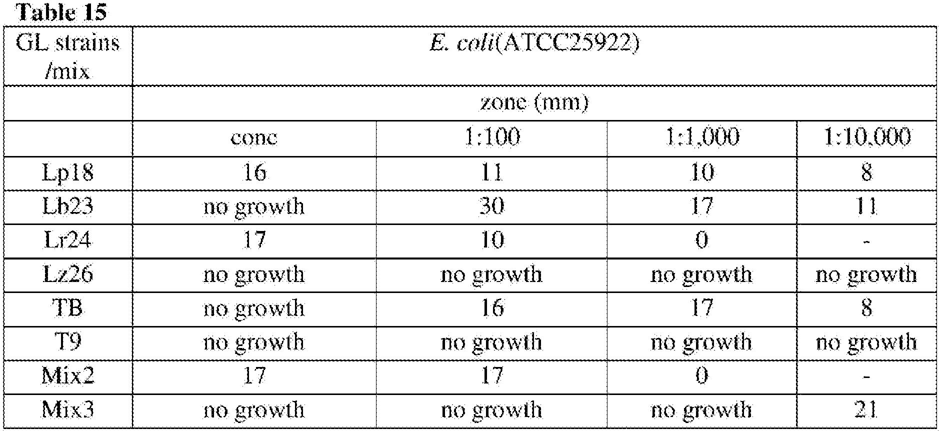

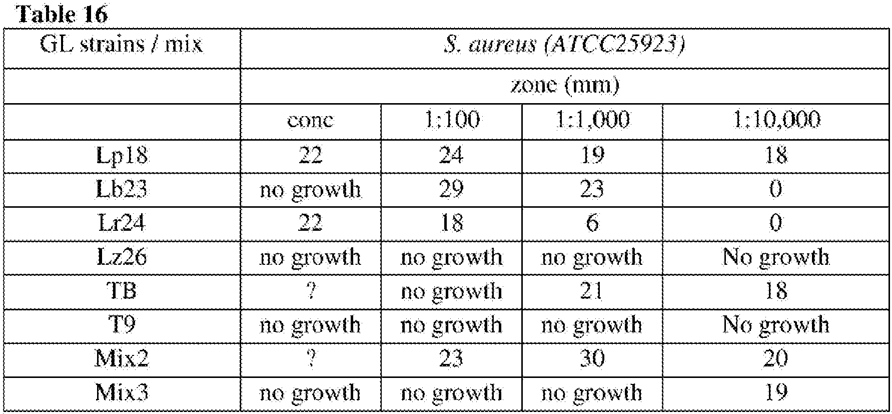

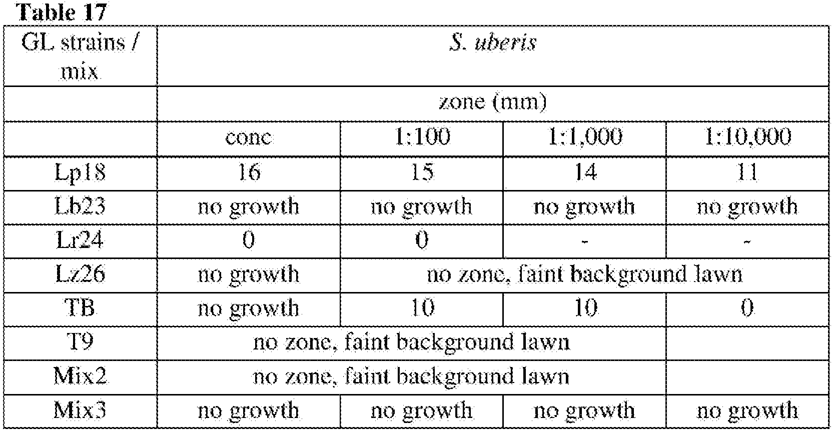

Zones of inhibition of growth (mm) of bacterial isolates 24 hours after addition of GL strains were also determined (as described in above examples). The results are shown in Tables 18, 19 and 20.

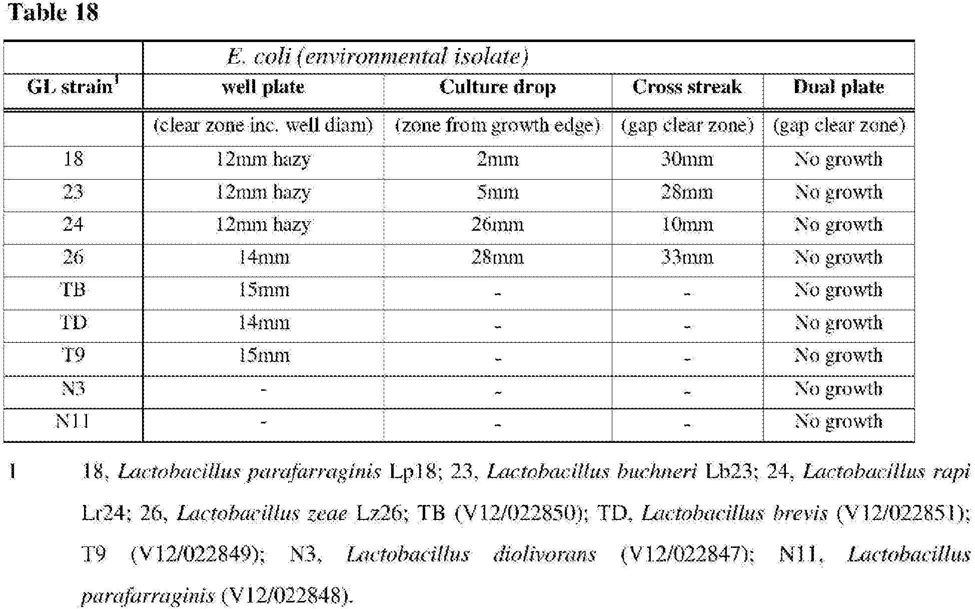

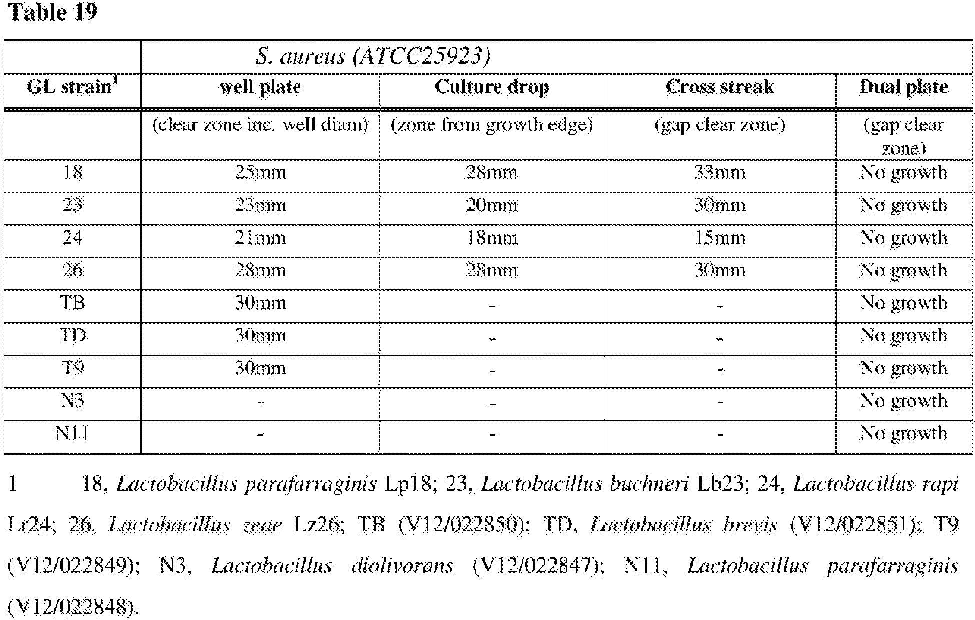

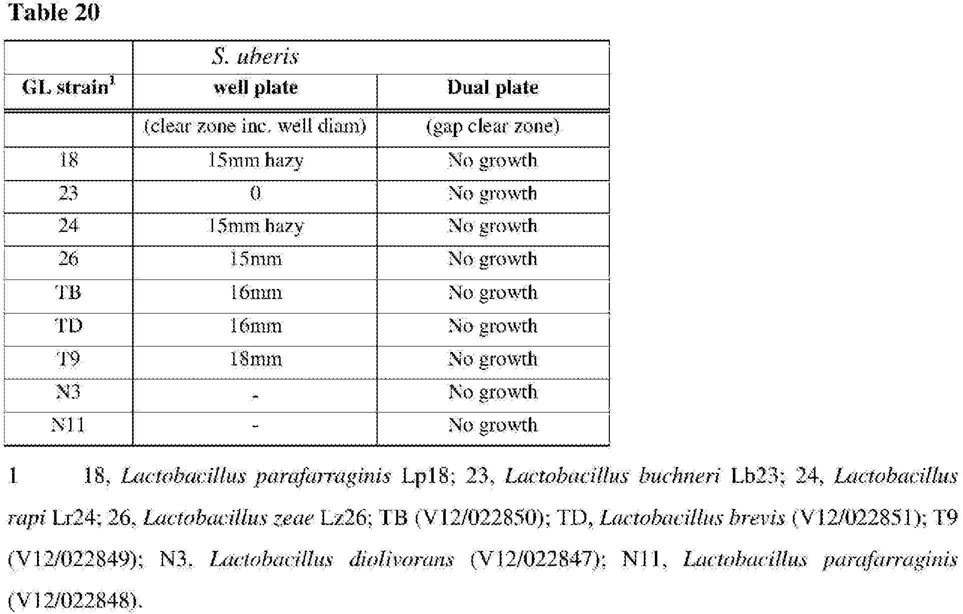

### Example 8- Activity against Pseudomonas savastanoi

The inventors also investigated the ability of GL strains described in Example 1 to inhibit growth of *Pseudomonas savastanoi* (causative agent of, *inter alia,* olive gall disease) using the dual plates screen method as described in Example 2. The results are shown in Table 21.

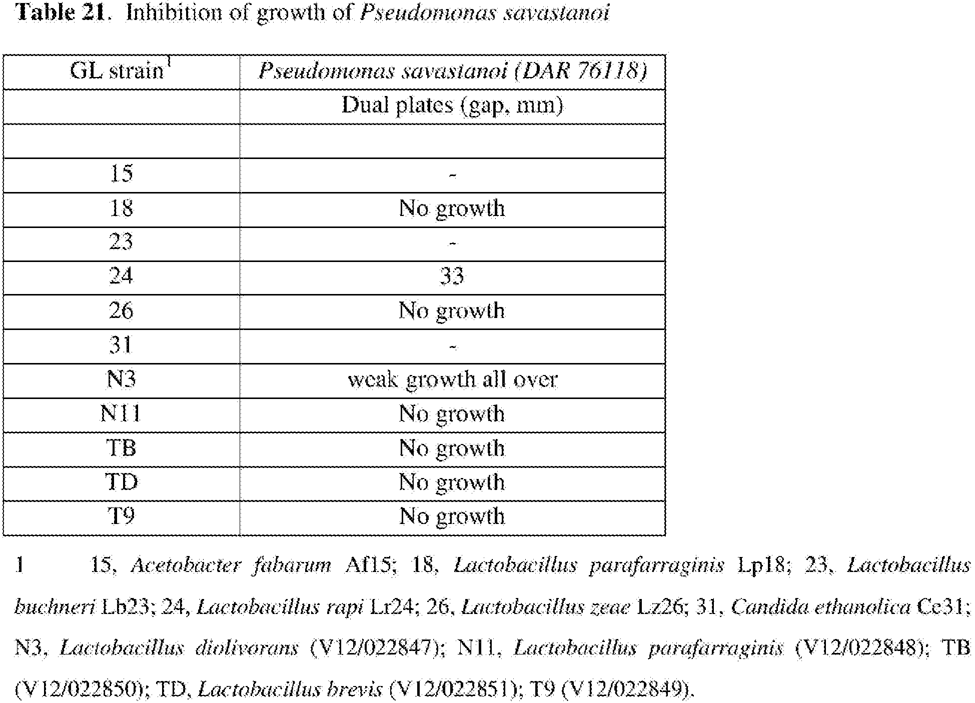

## Claims

1. A method of treating or preventing infection of a plant by a fungal or bacterial pathogen, the method comprising administering to the plant, or otherwise exposing the plant to, an effective amount of a composition comprising *Lactobacillus parafarraginis* strain Lp18 deposited with National Measurement Institute, Australia on 27 October 2011 under Accession Number V1 1/022945, or a culture supernatant or cell free filtrate derived from culture media in which said *Lactobacillus parafarraginis* strain has been cultured, wherein the fungal or bacterial pathogen is selected from *Pseudocercospora macadamiae, Botrytis cinerea, Streptomyces scabies,* and *Pseudomonas savastanoi.*

2. The method according to claim 1, wherein the *Lactobacillus parafarraginis* is encapsulated.

3. The method according to claim 1 or claim 2, wherein the plant is a crop species.

## Patentansprüche

1. Verfahren zur Behandlung oder Vorbeugung einer Infektion einer Pflanze durch ein fungales oder bakterielles Pathogen, wobei das Verfahren die Verabreichung an die Pflanze, oder eine anderweitige Exposition der Pflanze gegenüber, einer wirksamen Menge einer Zusammensetzung umfasst, die den Stamm von Lactobacillus parafarraginis Lp18 umfasst, der beim Nationalen Messinstitut in Australien am 27. Oktober 2011 unter der Zugangsnummer V11/022945 hinterlegt wurde, oder einen Kulturüberstand oder ein zellfreies Filtrat, das von Kulturmedien stammt, in denen der Stamm von Lactobacillus parafarraginis kultiviert worden ist, wobei das fungale oder bakterielle Pathogen ausgewählt ist aus Pseudocercospora macadamiae, Botrytis cinerea, Streptomyces scabies und Pseudomonas savastanoi.

2. Verfahren nach Anspruch 1, wobei der Lactobacillus parafarraginis verkapselt ist.

3. Verfahren nach Anspruch 1 oder 2, wobei die Pflanze eine Nutzpflanzenart ist.

## Revendications

1. Procédé de traitement ou de prévention de l'infection d'une plante par un agent pathogène fongique ou bactérien, le procédé comprenant l'administration à la plante, ou autrement l'exposition de la plante à, d'une quantité efficace d'une composition comprenant la souche Lp18 de *Lactobacillus parafarraginis* déposée auprès du National Measurement Institute, Australie le 27 octobre 2011 sous le numéro d'accès V11/022945, ou d'un surnageant de culture ou d'un filtrat acellulaire dérivé d'un milieu de culture dans lequel ladite souche de *Lactobacillus parafarraginis* a été cultivée, dans lequel l'agent pathogène fongique ou bactérien est choisi parmi *Pseudocercospora macadamiae, Botrytis cinerea, Streptomyces scabies* et *Pseudomonas savastanoi.*

2. Procédé selon la revendication 1, dans lequel le *Lactobacillus parafarraginis* est encapsulé.

3. Procédé selon la revendication 1 ou la revendication 2, dans lequel la plante est une espèce cultivée.
